# EUROPEAN PATENT APPLICATION

(11) **EP 2 251 026 A1**
(43) Date of publication of application: **17.11.2010**
(21) Application number: 10155244.6
(22) Date of filing: 08.06.2001
(51) Int. Cl.: A61K 38/00, A61K 38/02, A61K 38/17, A61K 39/395, G01N 33/53, C07K 2/00, C07K 14/00, C07K 14/435, C07K 16/18, C07K 16/28, C07H 21/02

(54) **Methods and compositions for inhibiting immunoglobulin-mediated reperfusion injury**

(30) Priority: 08.06.2000 US 210272 P
(62) Divisional of application: 01942082.7
(71) Applicant: Immune Disease Institute, Inc., Boston, MA 02115 (US); The President and Fellows of Harvard College, Cambridge, MA 02138 (US); The Brigham and Women's Hospital, Inc., Boston MA 02115 (US)
(72) Inventor: Carroll, Michael, Wellesley, MA 02482 (US); Moore Jnr, Francis, Medfield, MA 02052 (US); Hechtman, Herbert, Chestnut Hill, MA 02467 (US)
(74) Representative: Williams, Gareth Owen

(57) **Abstract**

Methods and compositions for treating or preventing immunoglobulin-mediated reperfusion or ischemic injury, in a subject, are provided. Methods for identifying inhibitors of an interaction between a pathogenic immunoglobulin and an ischemic antigen or a component of the complement pathway are provided. Pathogenic immunoglobulins and mutated forms thereof are also disclosed.

## Description

### Background of the Invention

The complement system participates in both innate and adaptive immunity (Muller-Eberhard, H.J. (1988) Ann. Rev. Biochem. 57, 321-347; Carroll, M.C. (1998) Ann. Rev. Immunol 16, 545-568; Fearon et al., (1995) Annu. Rev. Immunol 13, 127-149). Recent studies using mice deficient in specific components of serum complement or complement receptors (e.g., CD21/CD35) have not only confirmed many known roles for complement, but shed new light on mechanisms important in natural and adaptive immunity (Carroll, M.C. (1998) *supra*). For example, one mechanism by which complement enhances the generation of memory responses to T-dependent antigens is by mediating a survival signal in germinal center (GC) B cells via the CD21/CD19/Tapa-1 co-receptor. Alternatively, the deficient mice have been important in confirming the importance of identifying the classical pathway as being essential for induction of injury (Weiser et al., (1996) J. Exp. Med. 1857-1864).

Ischemia reperfusion (J/R) injury represents refers to inflammatory injury to the endothelium and underlying parenchymal tissues following reperfusion of hypoxic tissues. It is a general syndrome that is responsible for both acute and chronic injury to various tissues including, for example, myocardium, central nervous system, hind limb and intestine. Ischemia reperfusion injury can result in necrosis and irreversible cell injury. For example, reperfusion of ischemic skeletal muscle results in endothelial cell injury characterized by vascular leakage with permeability edema (Weiser et al., (1996) J. Exp. Med. 1857-1864), and small bowel reperfusion leads to destruction of the mucosa (Williams, et al., (1999) J. Appl. Physiol., 938-942). In general, the longer the period of ischemia the more pronounced the inflammatory response. The,extent of I/R injury is also determined by such factors as the size of the ischemic region and the particular tissues involved (Williams, et al., (1999) J. Appl. Physiol., 938-942).

A major mediator of I/R injury is the complement system. Weisman et al. reported a mechanism for partially blocking I/R injury using a soluble inhibitor of complement C3b (sCR1) in a rat cardiac model (Weisman et al. (1990) Science 249, 146-151). The soluble CR1 receptor is very effective in inactivating C3 convertase, i.e. the enzyme complex that converts native C3 to its active C3b form. The CR1 receptor serves both as a co-factor in factor I cleavage of C3b and in displacing C3b from the convertase. Intravenous injection of mice with sCR1 prior to induction of ischemia significantly reduced injury on reperfusion of the treated animals. Histologic examination of treated animals revealed a reduction in the level of complement deposition in cardiac muscle and in the extent of tissue injury (Weisman et al. (1990) *supra*). Similar results were obtained by pretreating mice with sCR1 in a hindlimb model of injury (Hill et al. (1992) J. Immunol. 149, 1723-1730). In this model, the blood flow to the lower hindlimb was blocked for approximately 2 hours by placing a tourniquet on the hindlimb. Prior to reestablishment of blood flow to the tissues, mice were administered radio-iodinated albumin intravenously as a marker for permeability.

While these studies determine that injury was mediated by complement they did not address the question of mechanism of initiation of injury. Weiser et al. have demonstrated that mice deficient in antibody are partially protected from injury in the hindlimb model. Injury is restored by reconstituting with normal mouse serum. Williams et al. extended this observation to the small intestinal model and demonstrated that purified IgM was sufficient to restore injury in the antibody-deficient mice.

### Summary of the Invention

The present invention is based, in part, on the identification of pathogenic immunoglobulins (Ig), in particular pathogenic IgMs, which recognize an ischemia-specific antigen. Without being bound by theory, it is believed that binding of these pathogenic IgMs to an ischemia-specific antigen triggers, *inter alia*, activation of the complement pathway, which ultimately results in reperfusion or ischemic injury. Applicants also discovered that the pathogenic IgMs are produced by a subpopulation of B cells, referred to herein as "B-1 cells".

Accordingly, the present invention features a method for treating or preventing immunoglobulin-mediated reperfusion or ischemic injury in a subject, comprising administering to the subject an effective amount of an inhibitor of an interaction between a pathogenic immunoglobulin, e.g., a pathogenic IgM, and an ischemia-specific antigen, thereby reducing the number of the pathogenic immunoglobulins bound to the ischemia-specific antigen, such that the injury is inhibited or reduced.

In a preferred embodiment, the reperfusion or ischemic injury results following a naturally occurring episode, e.g., as a stroke. The reperfusion or ischemic injury can occur during and/or following a surgical procedure. Exemplary surgical procedures that cause the injury include a vessel-corrective technique selected from the group consisting of angioplasty, stenting procedure, atherectomy, and bypass surgery. In a preferred embodiment, the reperfusion or ischemic injury occurs in a cardiovascular tissue, e.g., the heart.

Preferably, the inhibitor of the present invention antagonizes the pathogenic immunoglobulin by one or more of the following activities: (i) inhibits or reduces an interaction (e.g., binding) between the pathogenic immunoglobulin and the ischemia-specific antigen; (ii) inhibits or reduces an interaction (e.g., binding) between the pathogenic immunoglobulin and a component of the complement pathway; (iii) neutralizes the pathogenic immunoglobulin by, e.g., sequestering the immunoglobulin and/or targeting its degradation; or (iv) inhibits or reduces production of the pathogenic immunoglobulin, e.g., blocks synthesis, assembly, and/or posttranslational modifications of the pathogenic antibody. The inhibitor can be a protein or a peptide; a small molecule; an antibody or a fragment thereof, e.g., an anti-idiotypic antibody; a carbohydrate; or a glycoprotein. In another preferred embodiment, the inhibitor can be a modified antibody as described herein. In a preferred embodiment, the inhibitor is administered in one or in sequential exposures over a period of hours, days, weeks, months or years. The inhibitor may be administered prior to, during, and/or after a naturally occurring episode that causes the injury. The inhibitor can also be administered prior to, during, and/or after a surgical procedure e.g. a surgical procedure associated with reperfusion or ischemic injury. In a preferred embodiment, the inhibitor is administered in combination with other therapeutic agents, e.g., an anti-coagulation agent, a complement inhibitor.

In another aspect, the invention features a method for treating or preventing immunoglobulin-mediated reperfusion or ischemic injury, in a subject, comprising administering to the subject an effective amount of an inhibitor of an interaction between a pathogenic immunoglobulin, e.g., a pathogenic IgM, and a component of the complement pathway, thereby reducing the number of pathogenic immunoglobulins which activate complement activity, such that the injury is inhibited or reduced.

In a preferred embodiments, the pathogenic immunoglobulin is an IgM or an IgG (e.g., an IgG1 and IgG3). Preferably, the pathogenic immunoglobulin is an IgM, and more preferably, it is a subclass of IgMs. In another preferred embodiment, the pathogenic immunoglobulin is produced by a subpopulation ofB cells, for example, B-1 cells, or the pathogenic immunoglobulin is produced by the hybridoma which will be which will be deposited with the ATCC, having Accession Number__________ . The pathogenic immunoglobulin can have a light chain variable region comprising an amino acid sequence shown in SEQ ID NO:2, and a heavy chain variable region comprising an amino acid sequence shown in SEQ ID NO:8. In a preferred embodiment, the ischemia-specific antigen is present on the surface of an endothelial cell and/or parenchymal tissue.

In preferred embodiments, the complement pathway is a classical pathway of complement, and the component of the complement pathway can be a C1 molecule or a subunit thereof (e.g., C1q). In another embodiment, the subject is a mammal, e.g., a rodent (e.g., a mouse) or a primate (e.g., a human).

In a preferred embodiment, the reperfusion or ischemic injury results following a naturally occurring episode, e.g., as a stroke. The reperfusion or ischemic injury can occur during and/or following a surgical procedure. Exemplary surgical procedures that cause the injury include a vessel-corrective technique selected from the group consisting of angioplasty, stenting procedure, atherectomy, and bypass surgery. In a preferred embodiment, the reperfusion or ischemic injury occurs in a cardiovascular tissue, e.g., the heart.

Preferably, the inhibitor of the present invention antagonizes the pathogenic immunoglobulin by one or more of the following activities: (i) inhibits or reduces an interaction (e.g., binding) between the pathogenic immunoglobulin and the ischemia-specific antigen; (ii) inhibits or reduces an interaction (e.g., binding) between the pathogenic immunoglobulin and a component of the complement pathway; (iii) neutralizes the pathogenic immunoglobulin by, e.g., sequestering the immunoglobulin and/or targeting its degradation; or (iv) inhibits or reduces production of the pathogenic immunoglobulin, e.g., blocks synthesis, assembly, and/or posttranslational modifications of the pathogenic antibody. The inhibitor can be a protein or a peptide; a small molecule; an antibody or a fragment thereof, e.g., an anti-idiotypic antibody; a carbohydrate; or a glycoprotein. In another preferred embodiment, the inhibitor can be a modified antibody as described herein. In a preferred embodiment, the inhibitor is administered in one or in sequential exposures over a period of hours, days, weeks, months or years. The inhibitor may be administered prior to, during, and/or after a naturally occurring episode that causes the injury. The inhibitor can also be administered prior to, during, and/or after a surgical procedure e.g. a surgical procedure associated with reperfusion or ischemic injury. In a preferred embodiment, the inhibitor is administered in combination with other therapeutic agents, e.g., an anti-coagulation agent, a complement inhibitor.

In another aspect, the invention features, a method for treating or preventing immunoglobulin-mediated reperfusion or ischemic injury in a subject, comprising removing from the subject or inactivating a pathogenic immunoglobulin, e.g., a pathogenic IgM as described herein, and/or B cells producing the pathogenic immunoglobulin (e.g., B-1 cells as described herein), thereby reducing the amount of the pathogenic immunoglobulin and/or B cells present in the subject.

In a preferred embodiment, the removing or inactivating step is performed *ex vivo*. In one embodiment, the pathogenic immunoglobulins or B cells can be removed by hemoperfusion. In yet another embodiment, the B cells can be removed using a B cell-specific antibody (e.g., an anti-B-1 antibody or an anti-CD5 antibody or anti-CD11G/CD18). The pathogenic immunoglobulin, e.g., an IgM, can be removed by contacting blood from a subject with an immobilized antigen (e.g., an ischemia-specific antigen) or an immobilized anti-idiotypic antibody. In a preferred embodiment, the removing or inactivating step of the pathogenic immunoglobulin is performed by administering an anti-idiotypic antibody to the subject. In another embodiment, the removing or inactivating step of the B cell is performed by administering to the subject a B cell targeting moiety (e.g., an antibody or an antigen binding fragment thereof, or an antigen) coupled to a toxin, e.g., ricin or diphteria toxin. In a preferred embodiment, the subject is a mammal, e.g., a rodent (e.g., a mouse) or a primate (e.g., a human). In a preferred embodiment, the reperfusion or ischemic injury results following a naturally occurring episode, e.g., as a stroke. Preferably, the removing step is carried out within minutes, one to five hours, five to ten hours, ten to twenty hours, one to five days, following the naturally occurring episode. In a preferred embodiment, the reperfusion or ischemic injury occurs in a cardiovascular tissue, e.g., the heart. In another embodiment, the reperfusion or ischemic injury is prevented and/or decreased by, removing from the subject, the pathogenic immunoglobulin, and/or the B cells, prior to, during, and/or following the surgical procedure. For example, the removing step can be carried out at least one to five hours, five to ten hours, ten to twenty hours, or one, two or three days prior to the surgical procedure. The removing step can also be continued for appropriate time intervals during and after the surgical procedure.

In another aspect, the invention features an isolated pathogenic immunoglobulin, e.g., a pathogenic IgM as described herein. Preferably, the pathogenic immunoglobulin has one or more of the following properties: (i) is capable of interacting with an ischemia-specific antigen; (ii) is capable of fixing complement; or (iii) is produced by a subpopulation ofB cells. In a preferred embodiment, the pathogenic immunoglobulin is produced by a subpopulation ofB cells, e.g., B-1 cells. In another embodiment, the pathogenic immunoglobulin is produced by the hybridoma that will be deposited with the ATCC, having Accession Number___________ . The pathogenic immunoglobulin can have a light chain variable region comprising an amino acid sequence shown in SEQ ID NO:8. In another embodiment, the pathogenic immunoglobulin has a heavy chain variable region comprising an amino acid sequence shown in SEQ ID NO:2. In another embodiment, the pathogenic immunoglobulin interacts with (e.g., binds to) an ischemia-specific antigen that is present on the surface of an endothelial cell.

In another aspect, the invention features, a pathogenic immunoglobulin, e.g., a pathogenic IgM as described herein. In a preferred embodiment, the pathogenic immunoglobulin interacts with (e.g., binds to) an ischemia-specific antigen. The pathogenic immunoglobulin can feature a reduced ability to activate complement. In a preferred embodiment, the pathogenic immunoglobulin has one or more amino acid substitutions, deletions, and/or insertions. For example, one or more of the amino acid residues involved in complement binding and/or activation are mutated. In a preferred embodiment, the pathogenic immunoglobulin that is a pathogenic immunoglobulin produced by a cell line that will be deposited with the ATCC, having Accession Number _____________. In other embodiments, the pathogenic antibody, or antigen binding portion thereof, has a light chain variable region comprising the amino acid sequence of SEQ ID NO:8 or a fragment thereof In other embodiments, the pathogenic antibody comprises at least the CDR1 region of SEQ ID NO:10, or antigen binding portions thereof, and/or at least one CDR2 region of SEQ ID NO:12, or an antigen binding portion thereof. Preferably, the pathogenic antibody comprises at least the CDR1 region of SEQ ID NO:10, and the CDR2 region of SEQ ID NO:12, or antigen binding portions thereof. In another embodiment, the pathogenic antibody, or antigen binding portion thereof, has a heavy chain variable region comprising the amino acid sequence of SEQ ID NO:2 or a fragment thereof. In other embodiments, the pathogenic antibody comprises at least the CDR1 region of SEQ ID NO:4, or antigen binding portions thereof, and/or at least one CDR2 region of SEQ IID NO:6, or an antigen binding portion thereof Preferably, the pathogenic antibody comprises at least the CDR1 region of SEQ ID NO:4, and the CDR2 region of SEQ ID NO:6, or antigen binding portions thereof

In another embodiment, the pathogenic antibody, or antigen binding portion thereof, has a light chain variable region comprising the amino acid sequence shown in SEQ ID NO:8, or an antigen binding fragment thereof, and a heavy chain variable region comprising the amino acid sequence shown in SEQ ID NO:2, or an antigen binding fragment thereof The pathogenic antibody can be a human antibody having a binding affinity to the ischemia specific antigen, similar, e.g., greater than, less than, or equal to, the binding affinity of the pathogenic antibody produced by the hybridoma that will be deposited with the ATCC, having Accession Number ____________. In another embodiment, the pathogenic antibody can be a non-human antibody, e.g., a cow, goat, mouse, rat, sheep, pig or rabbit. Preferably, the non-human antibody is a murine antibody. The pathogenic antibody can be a recombinant antibody. In a preferred embodiment, the pathogenic antibody is a humanized antibody. In another embodiment, the isolated pathogenic immunoglobulin possesses the same antigenic specificity as the immunoglobulin produced by the hybridoma which will be deposited with the ATCC, having Accession Number _____________.

In another aspect, the invention features, a method for isolating an ischemia-specific antigen, comprising providing a pathogenic immunoglobulin, e.g., a pathogenic IgM, e.g., a pathogenic IgM described herein, contacting a sample containing the ischemia-specific antigen, e.g., a biochemical isolate (e.g., an endothelia cell isolate), or a collection of proteins (e.g., an expression library) with the pathogenic immunoglobulin under conditions that allow specific binding of the pathogenic immunoglobulin to the sample, detecting any changes in the level of binding of the pathogenic antibody to the sample relative to a control, wherein a change in the level of binding of the pathogenic immunoglobulin in the presence of the sample relative to that detected in the control is indicative of specific binding, and isolating, e.g., purifying, the ischemia-specific antigen from the sample. In a preferred embodiment, the sample is enriched in the ischemia-specific antigen, e.g., it is obtained from a subject (e.g., a human patient) with reperfusion or ischemic injury. In other embodiments, the sample is a biochemical isolate, e.g., an endothelial tissue, or an expression library, e.g., a library derived from an endothelial tissue. In a preferred embodiment, the contacting step occurs *in vitro.*

In another aspect, the invention features, a method for identifying an inhibitor of an interaction between a pathogenic immunoglobulin, e.g., a pathogenic IgM, and an ischemia-specific antigen from one or more (e.g., a plurality of) test compounds, comprising providing a reaction mixture which includes the pathogenic immunoglobulin and the ischemia-specific antigen (e.g., an endothelial tissue or lysate obtained from a subject (e.g., a human patient) with reperfusion or ischemic injury) under conditions that allow binding of the pathogenic immunoglobulin and the ischemia-specific antigen to occur, contacting the pathogenic immunoglobulin and the ischemia-specific antigen with one or more test compounds (e.g., members of a combinatorial library), and detecting any changes in binding of the pathogenic immunoglobulin and the ischemia-specific antigen in the presence of a given test compound relative to that detected in the absence of the test compound, wherein a change (e.g., decrease) in the level of binding between the pathogenic immunoglobulin and the ischemia-specific antigen in the presence of the test compound relative to that detected in the absence of the test compound indicates that the test compound is an inhibitor of the interaction between the pathogenic immunoglobulin and the ischemia-specific antigen. In a preferred embodiment, the contacting step is effected in vivo. The method can further include pre-treating the pathogenic immunoglobulins with one or more test compounds. The pre-treated pathogenic immunoglobulins can then be injected into mice deficient in pathogenic immunoglobulins.

In another aspect, the invention features, a method for identifying an inhibitor of an interaction between a pathogenic immunoglobulin, e.g., a pathogenic IgM, and component of the complement pathway from one or more (e.g., a plurality of) test compounds, comprising providing a reaction mixture which includes the pathogenic immunoglobulin and the component of the complement pathway under conditions that allow binding of the pathogenic immunoglobulin and the component of the complement pathway to occur, contacting the pathogenic immunoglobulin and the component of the complement pathway with one or more test compounds (e.g., members of a combinatorial library), and detecting any changes in binding of the pathogenic immunoglobulin and the component of the complement pathway in the presence of a given test compound relative to that detected in the absence of the test compound, wherein a change (e.g., decrease) in the level of binding between the pathogenic immunoglobulin and the component of the complement pathway in the presence of the test compound relative to that detected in the absence of the test compound indicates that the test compound is an inhibitor of the interaction between the pathogenic immunoglobulin and the component of the complement pathway. In preferred embodiments, the pathogenic immunoglobulin is a pathogenic IgM as described herein, and the ischemia-specific antigen is obtained from an endothelial tissue, or a lysate, obtained from a subject (e.g., a human patient) with reperfusion or ischemic injury). In another embodiment, the method is performed *in vitro*. In a preferred embodiment, either the pathogenic immunoglobulin or the ischemia-specific antigen (or both) is labeled with a detectable signal, e.g., fluorophores, colorimetric enzymes, radioisotopes, luminescent compounds, and the like. The method can further include repeating at least one step, e.g., the contacting step with a second or subsequent member or members of the library. In a preferred embodiment, a plurality of test compounds, e.g., library members, is tested. The plurality of test compounds, e.g., library members, can include at least 10, 10², 10³, 10⁴, 10⁵, 10⁶, 10⁷, or 10⁸ compounds. In a preferred embodiment, the plurality of test compounds, e.g., library members, share a structural or functional characteristic. The test compound can be a peptide or a small organic molecule. In another embodiment, the complement pathway is a classical pathway of complement. In a preferred embodiment, the component of the complement pathway is a C1 molecule or a subunit thereof (e.g., C1q).

In another aspect, the invention features an isolated nucleic acid encoding a pathogenic TgM antibody, or fragments thereof In preferred embodiments, the isolated nucleic acid encodes a light chain variable region of SEQ ID NO:8. In another preferred embodiment, the nucleic acid sequence encodes a light chain variable region comprising a CDR1 of SEQ ID NO:10, or an antigen binding portion thereof. In another preferred embodiment, the nucleic acid sequence encodes a light chain variable region comprising a CDR2 of SEQ ID NO:12, or an antigen binding portion thereof. In another preferred embodiment, the nucleic acid sequence encodes a light chain variable region comprising a CDR1 of SEQ ID NO:10, or an antigen binding portion thereof, and a CDR2 of SEQ ID NO:12, or an antigen binding portion thereof. In a preferred embodiment, the nucleic acid encodes a light chain variable region of murine origin, human origin, or a combination of murine and human amino acid sequences. For example, the nucleic acid can encode a light chain variable region comprising the CDR1 of SEQ ID NO:10 and/or the CDR2 of SEQ ID NO:12, and a human framework sequence.

In yet other preferred embodiments, the isolated nucleic acid encodes a heavy chain variable region of SEQ ID NO:2. In another preferred embodiment, the nucleic acid sequence encodes a heavy chain variable region comprising a CDR1 of SEQ ID NO:4, or an antigen binding portion thereof In another preferred embodiment, the nucleic acid sequence encodes a heavy chain variable region comprising a CDR2 of SEQ ID NO:6, or an antigen binding portion thereof. In another preferred embodiment, the nucleic acid sequence encodes a heavy chain variable region comprising a CDR1 of SEQ ID NO:4, or an antigen binding portion thereof, and a CDR2 of SEQ ID NO:6, or an antigen binding portion thereof. In a preferred embodiment, the nucleic acid encodes a heavy chain variable region of murine origin, human origin, or a combination of murine and human amino acid sequences. For example, the nucleic acid can encode a heavy chain variable region comprising the CDR1 of SEQ ID NO:4 and/or the CDR2 of SEQ ID NO:6, and a human framework sequence. In still another embodiment, the isolated nucleic acid encodes both a light chain variable region comprising the amino acid sequence of SEQ ID NO:8 and a heavy chain variable region comprising the amino acid sequence of SEQ ID NO: 2.

In another aspect, the invention features an isolated nucleic acid molecules and fragments thereof. In a preferred embodiment, the isolated nucleic acid molecule comprises the light chain nucleotide sequence of SEQ ID NO:7. In another embodiment, the nucleic acid molecule comprises the light chain CDR1 nucleotide sequence of SEQ ID NO:9, or a portion thereof. In another preferred embodiment, the nucleic acid molecule comprises the light chain CDR2 nucleotide sequence of SEQ ID NO:11, or a portion thereof. In another preferred embodiment, the nucleic acid molecule comprises a light chain CDR1 nucleotide sequence of SEQ ID NO:9, or portion thereof, and a light chain CDR2 nucleotide sequence of SEQ ID NO:11, or portion thereof. The nucleic acid molecules of the present invention comprising light chain sequences, e.g. SEQ ID NO:7, SEQ ID NO: 9, SEQ ID NO:11, or combinations thereof, encompass nucleotides having 70%, 80%, 90%, 95%, 96%, 97%, 98%, and 99% sequence identity thereto. Further, the nucleic acid molecules of the present invention comprising light chain sequences, e.g. SEQ ID NO:7, SEQ ID NO: 9, SEQ ID NO:11, or combinations thereof, encompass nucleotides which hybridize under stringent conditions, e.g. low, medium, high or very high stringency conditions, thereto.

In another embodiment, the invention features nucleic acid molecules having at least 70%, 80%, 90%, 95%, 96%, 97%, 98%, and 99% sequence identity with a nucleic acid molecule encoding a light chain polypeptide, e.g. a light chain polypeptide of SEQ ID NO:8, SEQ ID NO: 10, and SEQ ID NO:12. In another embodiment, the invention features nucleic acid molecules which hybridize to a nucleic acid sequence encoding a light chain variable region of a pathogenic antibody or portion thereof, e.g., a light chain variable region of SEQ ID NO:8, SEQ ID NO: 10, and SEQ ID NO:12.

In yet other preferred embodiments, the nucleic acid molecules comprise the heavy chain nucleotide sequence of SEQ ID NO:1. In another embodiment, the nucleic acid molecule comprises the heavy chain CDR1 nucleotide sequence of SEQ ID NO:3, or a portion thereof. In another preferred embodiment, the nucleic acid molecule comprises the heavy chain CDR2 nucleotide sequence of SEQ ID NO:5, or a portion thereof. In another preferred embodiment, the nucleic acid molecule comprises a heavy chain CDR1 nucleotide sequence of SEQ ID NO:3, or a portion thereof, and a heavy chain CDR2 nucleotide sequence of SEQ ID NO:5, or a portion thereof. The nucleic acid molecules of the present invention comprising heavy chain sequences, e.g. SEQ ID NO:1, SEQ ID NO: 3, SEQ ID NO:5, or combinations thereof, also encompasses nucleotides having 70%, 80%, 90%, 95%, 96%, 97%, 98%, and 99% sequence identity thereto. Further, the nucleic acid molecules of the present invention comprising light chain sequences, e.g. SEQ ID NO:1, SEQ ID NO: 3, SEQ ID NO:5, or combinations thereof, encompass nucleotides which hybridize under stringent conditions, e.g. low, medium, high or very high stringency conditions thereto.

In another embodiment, the invention features nucleic acid molecules having at least 70%, 80%, 90%, 95%, 96%, 97%, 98%, and 99% sequence identity with a nucleic acid molecule encoding a heavy chain polypeptide, e.g. a heavy chain polypeptide of SEQ ID NO:2, SEQ ID NO: 4, and SEQ ID NO:6. In another embodiment, the invention features nucleic acid molecules which hybridize to a nucleic acid sequence encoding a heavy chain variable region of a pathogenic antibody or portion thereof, e.g., a heavy chain variable region of SEQ ID NO:2, SEQ ID NO: 4, and SEQ ID NO:6.

In another aspect the invention features isolated polypeptides and fragments thereof. In preferred embodiments, the isolated polypeptides comprise, for example, the amino acid sequences of SEQ ID NO:8, SEQ ID NO: 10, or SEQ ID NO: 12, or fragments or combinations thereof; or SEQ ID NO:2, SEQ ID NO:4, or SEQ ID NO:6, or fragments or combinations thereof. The polypeptides of the present invention include polypeptides having at least, but not more than, 20, 10, 5, 4, 3, 2, or 1 amino acids that differ from SEQ ID NO:8, SEQ ID NO:10, or SEQ ID NO:12; or SEQ ID NO:2, SEQ ID NO:4, or SEQ ID NO:6. Preferred polypeptides are polypeptides that retain biological activity, e.g., the ability to bind an eschemia-specific antigen, and/or the ability to bind complement. In another embodiment, the polypeptides comprise polypeptides having at least 70%, 80%, 90%, 95%, 96%, 97%, 98%, and 99% sequence identity with a light chain variable region, or portion thereof, e.g. a light chain variable region polypeptide of SEQ ID NO:8, SEQ ID NO:10, or SEQ ID NO:12. In another embodiment, the polypeptides comprise polypeptides having at least 70%, 80%, 90%, 95%, 96%, 97%, 98%, and 99% sequence identity with a heavy chain variable region, or portion thereof, e.g. a heavy chain variable region polypeptide of SEQ ID NO:2, SEQ ID NO:4, or SEQ ID NO:6. In another embodiment, the invention features a polypeptide comprising the amino acid sequence of SEQ ID NO:8 and SEQ ID NO:2, further comprising an IRES sequence.

In another aspect, the invention provides an expression vector comprising the nucleic acid molecules of the present invention, which encode SEQ ID N0:8, SEQ ID NO: 10:, or SEQ ID NO:12, or fragments or combinations thereof; or SEQ ID NO:2, SEQ ID NO:4, or SEQ ID NO:6 or fragments or combinations thereof. In a preferred embodiment, the expression vector comprises a nucleic acid molecule encoding an antibody light chain having a variable region comprising the amino acid sequence of SEQ ID NO: 8, or a portion thereof, and a nucleotide sequence encoding an antibody heavy chain having a variable region comprising the amino acid sequence of SEQ ID NO: 2, or a portion thereof. In another aspect, the invention provides a host cell comprising an expression vector of the present invention. In preferred embodiments, the host cell can include at least 1 or 2 or more expression vectors, i.e. one expression vector encoding a light chain, e.g. SEQ ID NO:8 or a portion thereof, and one expression vector encoding a heavy chain, e.g. SEQ ID NO:2 or a portion thereof. In another embodiment, the host cell can comprise an expression vector cable of expressing both the light chain variable region and the heavy chain variable region. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

Also within the scope of the invention are inhibitors of the pathogenic immunoblobulin/ischemic antigen, and/or pathogenic immunoglobulin/complement component interaction identified using the methods described herein.

### Brief Description of the Drawings

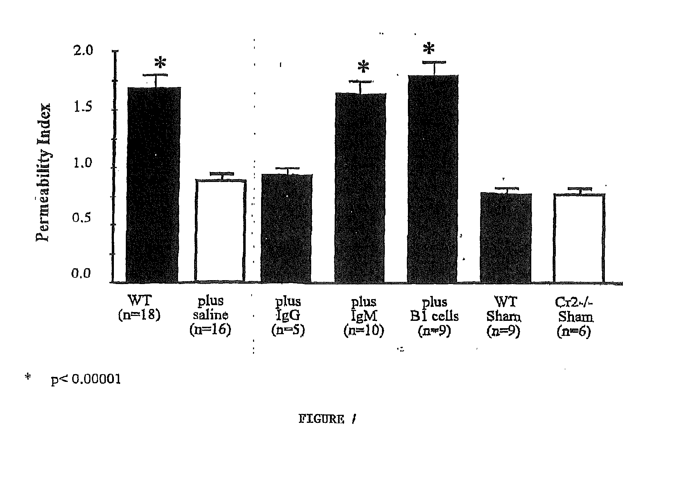

*Figure 1* is a bar graph depicting changes in intestinal permeability of inbred mice after intestinal ischemia and reperfusion or no injury (sham). WT represents parent strain for Cr2-/- mice. Cr2-/- was reconstituted with pooled IgG or IgM or saline control. Pooled IgM or IgG (0.5 mg) was administered intravenously approximately 1 hour before treatment. Values are means + standard error; n=number of mice in experimental groups.

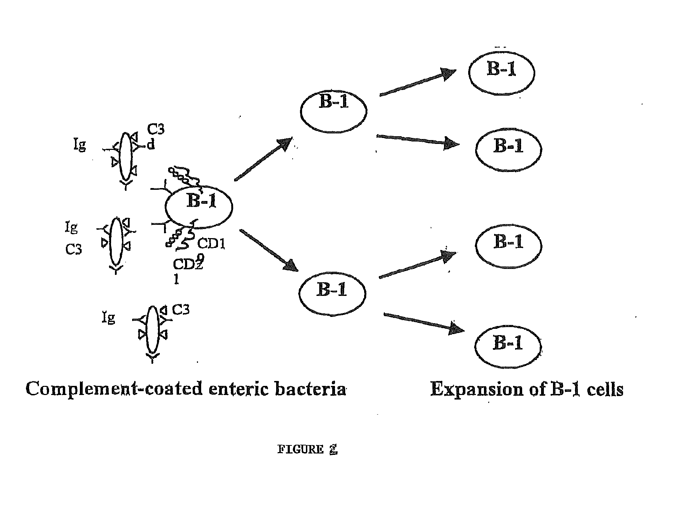

*Figure 2* is a schematic diagram of the proposed role for complement and complement receptors in positive selection of peritoneal B-1 lymphocytes.

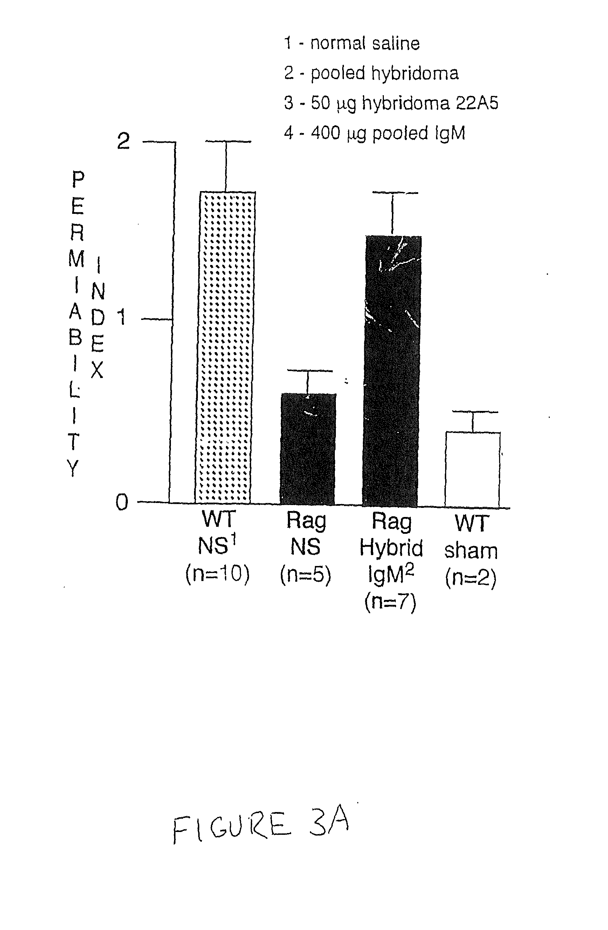

*Figure 3A* demonstrates reconstitution of I/R injury in antibody deficient mice (RAG-1) by pooled IgM from 22 individual B-1 cell hybridoma clones. 24 µg of each hybridoma IgM was pooled and injected intravenously 30 minutes before initial laparotomy. At the end of reperfusion, blood is obtained and permeability index is calculated as the ratio of ¹²⁵I counts of dried intestine versus that of blood. Values represent means ± SE; n = numbers of mice used in experimental groups. 1 = normal saline; 2 = pooled hybridoma; 3 = 50 µg hybridoma 22A5; 4 = 400 µg pooled IgM.

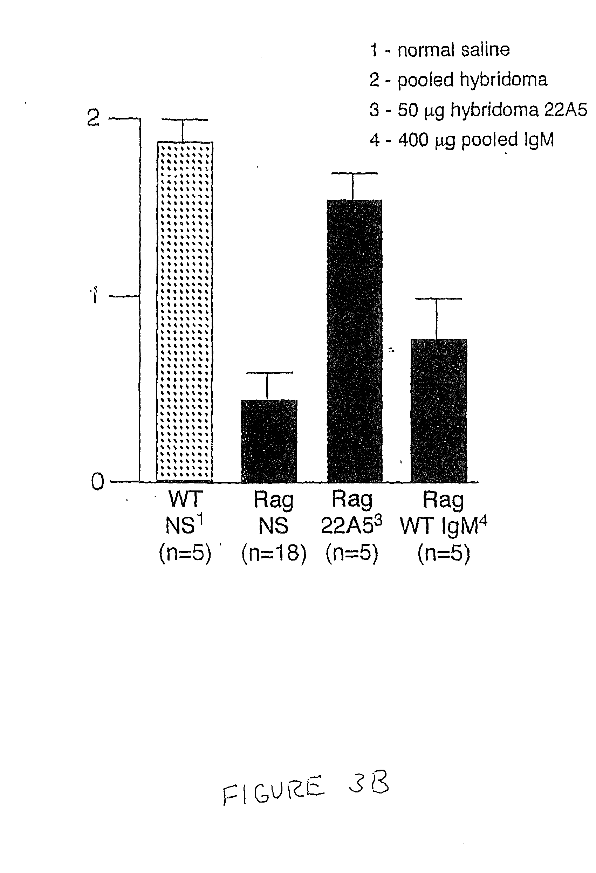

*Figure 3B* demonstrates that IgM from hybridoma clone 22A5 restores I/R injury in RAG-1 mice. Mice reconstituted with IgM receive either total serum IgM (400 µg) or 22A5 hybridoma IgM (50 µg each). Values represent means ± SE; n = numbers of mice used in experimental groups. 1 = normal saline; 2 = pooled hybridoma; 3 = 50 µg hybridoma 22A5; 4 = 400 µg pooled IgM.

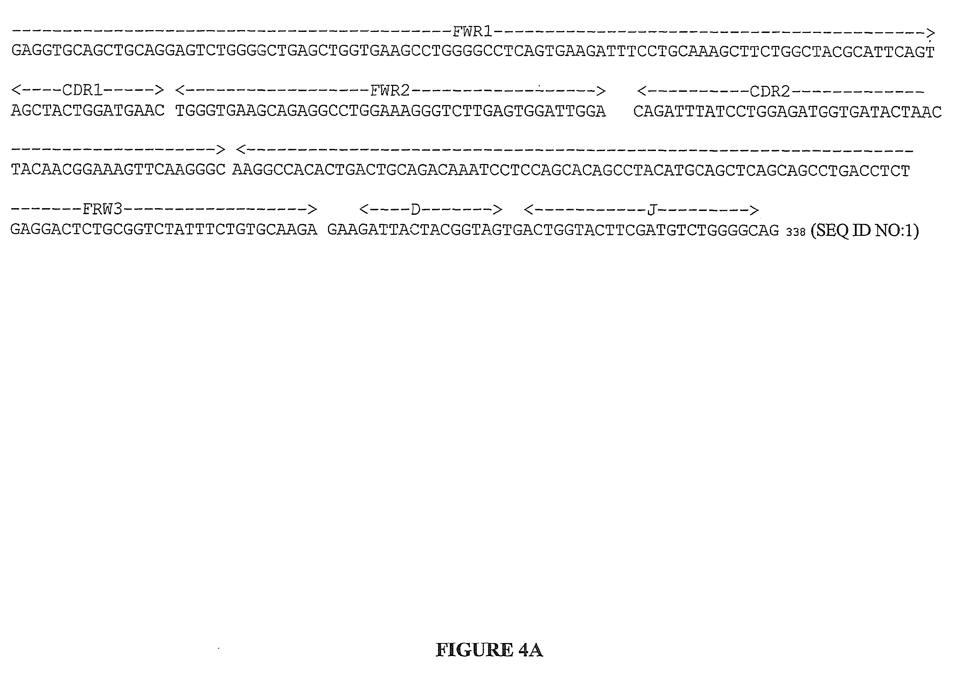

*Figure 4A* shows an IgM sequence analysis of B-1 hybridoma 22A5. The 22A5 IgM heavy chain nucleic acid sequence is shown (SEQ ID NO:1). RNA was purified from 22A5 hybridoma cells and reverse-transcribed into cDNA Semi-nested PCR using oligonucleotide primer specific for 5' VH and 3' Cm was used to amplify immunoglobulin heavy chain cDNAs, cDNA products were purified on agarose gels and the nucleotide sequence determined by automated sequencing. Framework regions (FWR) and complementarity-determining regions (CDR) are indicated above the nucleotides. A search of NCBI Nucleotide database found 22A5 heavy chain shared 95% homology with the immunoglobulin sequence VMU-3.2 (Accession number X03088).

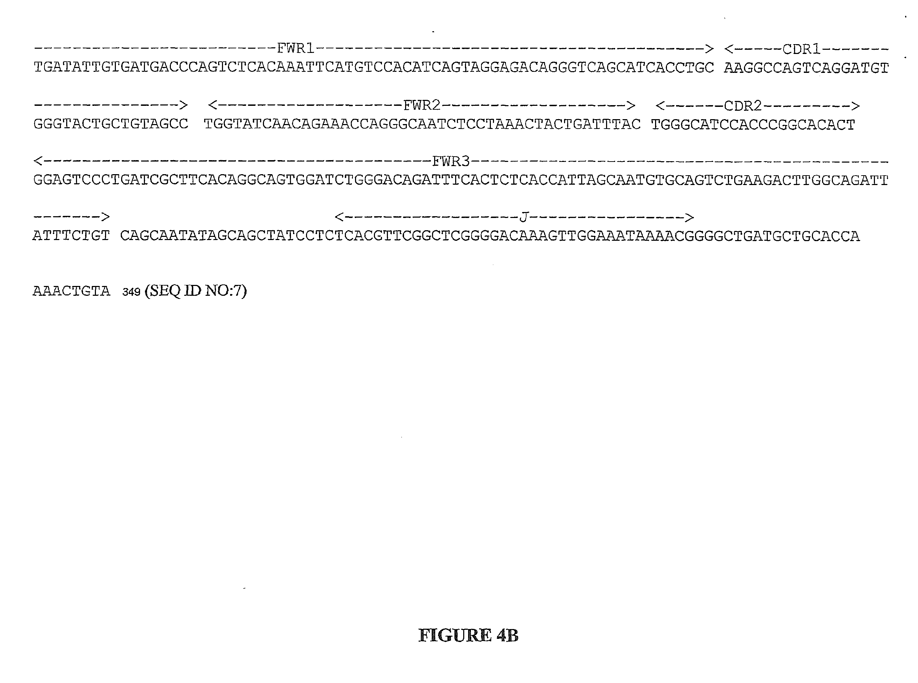

*Figure 4B* shows an IgM sequence analysis of B-1 hybridoma 22A5. The 22A5 IgM light chain nucleic acid sequence is shown (SEQ ID NO:7). RNA was purified from 22A5 hybridoma cells and reverse-transcribed into cDNA. Semi-nested PCR using oligonucleotide primer specific for 5' Vk and 3' Ck was used to amplify immunoglobulin light chain cDNAs. cDNA products were purified on agarose gels and the nucleotide sequence determined by automated sequencing. Framework regions (FWR) and complementarity-determining regions (CDR) are indicated above the nucleotides. A search of the NCBI Nucleotide database found 22A5 light chain shared homology with the immunoglobulin sequence Vk 19-23 gene (Accession number AJ23 5961).

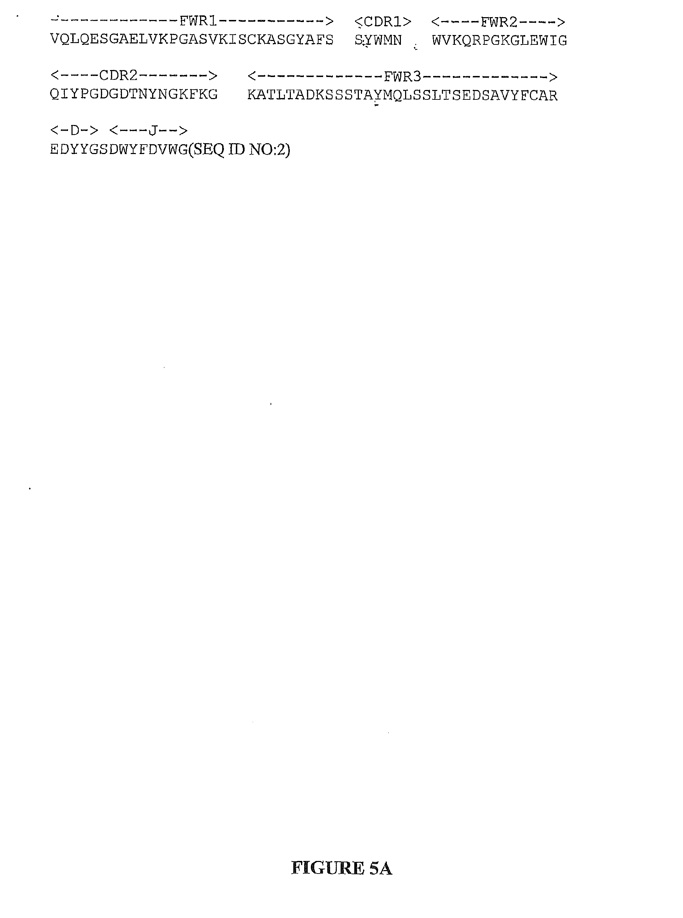

*Figure 5A* shows the amino acid sequence corresponding to the heavy chain nucleic acid sequence of SEQ ID NO: 1 (SEQ ID NO:2). Framework regions (FWR) and complementarity-determining regions (CDR) are indicated above the amino acid residues.

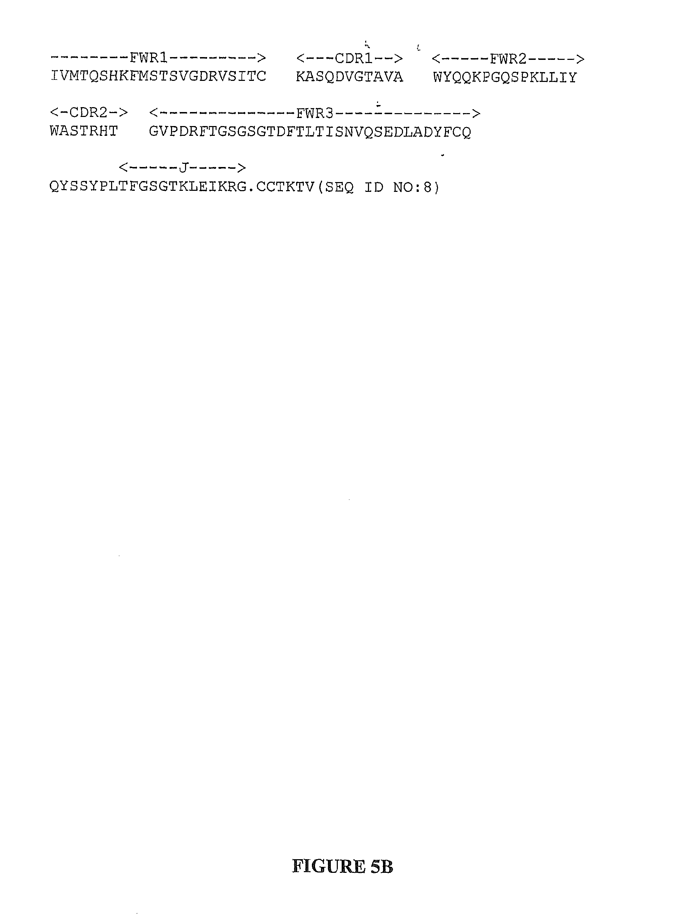

*Figure 5B* shows the amino acid sequence corresponding to the light chain nucleic acid sequence of SEQ ID NO:7 (SEQ ID NO:8). Framework regions (FWR) and complementarity-determining regions (CDR) are indicated above the amino acid residues.

### Detailed Description of the Invention

The present invention is based, in part, on the identification of pathogenic immunoglobulins (Ig), in particular pathogenic IgMs, which recognize an ischemia-specific antigen. It is believed that binding of these pathogenic IgMs to an ischemia-specific antigen triggers, *inter alia*, activation of the complement pathway, which ultimately results in reperfusion or ischemic injury. Applicants also discovered that the pathogenic IgMs are produced by a subpopulation of B cells, referred to herein as "B-1 cells". Accordingly, the present invention provides methods and compositions useful for treating or preventing, in a subject, tissue damage following reperfusion caused by a pathogenic immunoglobulin, e.g., a pathogenic IgMs.

Before further description of the invention, certain terms employed in the specification, examples and appended claims are, for convenience, collected here.

The terms "antibody" and "immunoglobulin" are used interchangeably herein.

As used herein, the terms "immunoglobulin" (or "Ig" in abbreviated form) and "antibody" refer to a glycoprotein comprising at least two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3, or four domains in the case of IgM, i.e. CH1, CH2, CH3, and CH4. Each light chain is comprised of a light chain variable region and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

There are up to five major chain heavy chain types, termed α, δ, ε, γ and µ, with g chains being further subdivided into γ1, γ2, γ3 and γ4 and the α chains into α1 and α2.
Two alternative light chain types are denoted κ and λ. The presence of the heavy chain gives rise to the major classes of immunoglobulins: IgA, IgD, IgE, IgG and IgM. The IgG can be further subdivided into the IgG1, IgG2, IgG3 and IgG4 subclasses, and IgA into the IgA1 and IgA2 subclasses.

As used herein, the term "immunoglobulin M" or "IgM" refers to a pentamer of five IgM monomers joined by the J chain. Monomeric IgM molecules have the same basic structure as the immunoglobulin molecules described above. IgM is the first immunoglobulin synthesized in an antibody response. As a pentamer, it has multiple functional domains and is, therefore, a potent activator of complement.

The term "pathogenic immunoglobulin" refers to an immunoglobulin molecule as described above that mediates tissue damage following reperfusion. In one embodiment, the pathogenic immunoglobulin may localize to an endothelial surface or parenchymal tissue, e.g., by binding to an antigen (e.g., an ischemic specific antigen) present on an endothelial cell surface or parenchymal tissue. Once localized to the endothelial surface or parenchymal tissue surface, the pathogenic immunoglobulin may mediate the binding of immune cells (e.g., effector cells) or a component of the classical complement system, e.g., Clq, thus triggering tissue damage. The antibodies can be of the various isotypes, including: IgG (e.g., IgG1, IgG2, IgG3, IgG4), IgM, IgA1, IgA2, IgA.sub.sec, IgD, of IgE. Preferably, the pathogenic immunoglobulin is an antibody isotype that is an activator of complement. Preferably, the pathogenic immunoglobulin is an IgM. The pathogenic immunoglobulins are preferably full-length (e.g., an IgM or IgG (e.g., an IgG1 and IgG3) antibody).

The term "antigen-binding portion" of an antibody (or simply "antibody portion" or "fragment"), as used herein, refers to one or more fragments of an antibody that retain the ability to specifically bind to an antigen (e.g. a target antigen, e.g., an ischemic antigen). Examples of binding fragments encompassed within the term "antigen-binding portion" of an antibody include (i) a Fab fragment, a monovalent fragment consisting of the VL, VH, CL and CH1 domains; (*ii*) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Manure 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

As used herein, "reperfusion" refers to the process whereby blood flow in the blood vessels is resumed after constriction or obstruction of flow, as in, e.g., ischemia.

The term "ischemic reperfusion" refers to an acute inflammatory response that follows reperfusion of ischemic tissues. Reperfusion may result following a naturally occurring episode, such as a stroke, or during a surgical procedure where blood flow in vessels is purposely blocked off. The constriction or occlusion of a blood vessel may be triggered as a result of, e.g., a blood clot, which blocks the blood flow in the vessel. Under circumstances such as these, endothelial integrity is disrupted and, as a result, may begin the coagulation cascade and new clots form which lodge in smaller capillaries downstream from the original clot obstruction. It is during this reflow period that tissue damage occurs.

"Immunoglobulin-mediated reperfusion injury" refers to a destruction in endothelial integrity that is mediated directly or indirectly by an immunoglobulin, e.g., a pathogenic immunoglobulin. Without being bound by theory, it is believed that during a period of ischemia, new antigens (e.g., ischemic specific antigens) are either expressed or exposed on the endothelial cell surface. These new antigens are then recognized by pathogenic immunoglobulins that accumulate near the site of injury. The localized pathogenic immunoglobulins are capable of triggering endothelial tissue damage, e.g., by activation of effector cell function or complement pathways.

As used herein, the term "ischemia" refers to a temporary or permanent deficiency of blood flow to an organ or tissue. In cardiovascular tissues, ischemia can give rise to at least two cardiac diseases -angina pectoris and acute myocardial infarction (heart attack). Typically, ischemia involving the heart muscle may result from coronary arterial disorders, such as atherosclerosis or spasms. The term "ischemic injury" refers to a destruction in endothelial integrity in, e.g., a tissue or organ, that occurs from a temporary or permanent deficiency of blood flow to the tissue or organ.

As used herein, the term "ischemia-specific antigen" refers to an antigen present on the surface of an endothelial cell. Preferably, the ischemia-specific antigen is expressed or exposed in response to an insult, e.g., an ischemic insult.

As used herein, "B-1 cells" refers to a subpopulation of B cells that produce the pathogenic immunoglobulins. Typically, B-1 cells express low levels of IgD and CD23, and a major fraction of these cells express the cell surface protein CD5 (Hardy et al. (1994) Immunol. Rev. 137, 91; Kantor et al. (1993) Annu. Rev. Immunol. 11, 501-538). They typically home to the peritoneal and mesenteric tissues and at least in mice do not appear to circulate (Herzenberg et al., (1986) Immunol. Rev. 93: 81-102; Martin et al., Cur Op. Immunol. 13: 195-201). Unlike conventional B-cells, they do not undergo somatic hypermutation and affinity maturation. They appear to arise during fetal and neonatal development and are not readily regenerated by adult bone marrow engraftment. These cells can further be distinguished by limited frequency in the peripheral lymph nodes and spleen, and by being primarily localized in the peritoneal cavity.

The term "complement" refers to a protein cascade composed of more than 20 proteins (including regulatory factors). Like the blood clotting system, activation of the system initiates a cascade of events in which proenzymes are sequentially activated proteolytically generating ligands for cell surface receptors, potent bioactive peptides and an end stage complex that intercalates in the membranes of cells leading to cell death. The central component is the third component or C3. It can be activated by three different pathways, i.e. classical, lectin or alternative (Reid et al., (1981) Semin. Immunopathol. 15:307-326; Muller-Eberhard, (1988) Ann. Rev. Biochem. 57: 321-347).

In one embodiment, the Pathogenic immunoglobulin may act via the classical complement pathway. The classical pathway is activated by an interaction between an antigen and antibody, which forms an immune complex. Antibodies can bind to, or "fix" complement only after reacting with their antigen. The formation of the complex provokes a conformational change in the antibody molecule that exposes a site for binding of the first complement component C1. C1 is a multimeric compound composed of six chains of three polypeptides each, termed Clq, and two each of C1s and C1r. Six C1q chains arrange themselves in a "bunch" and the four C1r and C1s molecules attach in a calcium-dependent interaction. When an antibody binds to two or more heads of C1q, C1r is cleaved to give an active molecule C1r, which cleaves C1s. C1s extends the activation process by cleaving the next complement component C4 to C4b, which continues the reaction process, and C4a. Cleavage of C4 to C4b reveals an internal thioester bond, which is swiftly inactivated by binding water molecules unless it can form covalent bonds with cell surface proteins or carbohydrates. If this occurs, C4b becomes relatively stable and binds to C2 in a magnesium-dependent reaction. C2 is then cleaved by C1s to form the complex C4b2a, known as the classical pathway C3 convertase. C3 is a similar molecule to C4, having an internal thioester bond. Two fragments derive from C3 cleavage. The smaller of these, C3a, has powerful biological properties. The larger C3b displays the labile binding site that allows the molecule to bind to C4b2a. The binding of C3 b to C4b2a leads to the generation of the last enzyme of the classical pathway, C4b2a3b, known as the classical pathway C5 convertase, which cleaves C5, a component of the membrane attack pathway.

In another embodiment, the pathogenic immunoglobulin may act via the alternative complement pathway. In the alternative pathway, C3 generates C3b, C3bB and C3bBb, which in turn cleaves C3. This process is accelerated if the active enzymes are stabilized on bacterial cell walls, or if more C3b is produced from the classical pathway. The alternative pathway C5 convertase C3bBb3b is generated.

As used herein, the language "subject" is intended to include human and non-human animals. Preferred human animals include a human patient with ischemic reperfusion injury. The term "non-human animals" or "non-human" of the invention includes all vertebrates, e.g., mammals and non-mammals, such as non-human primates, rodents, sheep, dog, cow, chickens, amphibians, reptiles, etc.

As used herein, an "inhibitor" refers to an agent that reduces or blocks (completely or partially) an interaction between a pathogenic immunoglobulin and an ischemia-specific antigen; or reduces or blocks an interaction between a pathogenic immunoglobulin and a component of the complement pathway, e.g., C1q. The term "interaction" refers to a physical association between two or more molecules, e.g., binding. The interaction may be direct or indirect. Preferably, the inhibitor antagonizes one or more of the following activities of the pathogenic immunoglobulin: (i) inhibits or reduces an interaction (e.g., binding) between the pathogenic immunoglobulin and the ischemia-specific antigen; (ii) inhibits or reduces an interaction (e.g., binding) between the pathogenic immunoglobulin and a component of the complement pathway, e.g., Clq; (iii) neutralizes the pathogenic immunoglobulin by, e.g., sequestering the immunoglobulin and/or targeting its degradation; or (iv) inhibits or reduces production of the pathogenic immunoglobulin, e.g., blocks synthesis, assembly, and/or posttranslational modifications of the pathogenic antibody. The inhibitor can be a protein (e.g., an antibody, e.g., an anti-idiotypic antibody or a fragment thereof), a peptide, or a small organic molecule. The terms "inhibitors" and "agents" are used interchangeably herein.

As used herein, an "effective amount" of an inhibitor refers to an amount of an agent which is effective, upon single- or multiple-dose administration to the subject, e.g., a patient, at reducing or eliminating reperfusion or ischemic injury. Such reduction or elimination of reperfusion injury can be reflected as a prolongation of the survival of the subject, e.g., a patient beyond that expected in the absence of such treatment, or any improvement in the prognosis of the subject relative to the absence of such treatment.
The inhibitor can be used to treat (e.g., reduce or eliminate an existing injury) or to prevent (e.g., delaying the occurrence of the onset or recurrence of) a reperfusion or ischemic injury. The skilled artisan will appreciate that certain factors influence dosage and timing required to effectively treat a subject, including but not limited to the severity of the disease or disorder, previous treatments, the general health and/or age of the subject, and other diseases present.

As used herein, the term "consensus sequence" refers to the sequence formed from the most frequently occurring amino acids (or nucleotides) in a family of related sequences (See e.g., Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0,2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").
The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm ofE. Meyers and W. Miller ((1989) CABIOS, 4:11-17) which has been incorporated into the ALIGN program (version 2.0), using a PAM120 weight residue table, a gap length penalty of 12 and a gap penalty of 4.

### Methods for Identifying Pathogenic Immunoglobulin Inhibitors

In one aspect, the present invention provides a method for identifying an inhibitor of an interaction between a pathogenic immunoglobulin and an ischemia-specific antigen, or a pathogenic immunoglobulin and a component of the complement pathway from one or more (e.g., a plurality of) test compounds. The inhibitor can be a protein (e.g., antibody or a fragment thereof); a peptide; a carbohydrate; a glycoprotein; or a small organic molecule.

### Libraries of Test Compounds

The inhibitor can be a small organic molecule, e.g., a member of a combinatorial library.

In one embodiment, the invention provides libraries of inhibitors. The synthesis of combinatorial libraries is well known in the art and has been reviewed (see, e.g., E.M. Gordon et al., J: Med. Chem. (1994) 37:1385-1401; DeWitt, S. H.; Czarnik, A. W. Acc. Chem. Res. (1996) 29:114; Armstrong, R. W.; Combs, A. P.; Tempest, P. A.; Brown, S. D.; Keating, T. A. Acc. Chem. Res. (1996) 29:123; Ellman, J. A. Acc. Chem. Res. (1996) 29:132; Gordon, E. M.; Gallop, M. A.; Patel, D. V. Acc. Chem. Res. (1996) 29:144; Lowe, G. Chem. Soc. Rev. (1995) 309, Blondelle et al. Trends Anal. Chem. (1995) 14:83; Chen et al. J. Am. Chem. Soc. (1994) 116:2661; U.S. PAtents 5,359,115, 5,362,899, and 5,288,514; PCT Publication Nos. WO92/10092, WO93/09668, WO91/07087, WO93/20242, WO94/08051).

Libraries of compounds of the invention can be prepared according to a variety of methods, some of which are known in the art. For example, a "split-pool" strategy can be implemented in the following way: beads of a functionalized polymeric support are placed in a plurality of reaction vessels; a variety of polymeric supports suitable for solid-phase peptide synthesis are known, and some are commercially available (for examples, see, e.g., M. Bodansky "Principles of Peptide Synthesis", 2nd edition, Springer-Verlag, Berlin (1993)). To each aliquot of beads is added a solution of a different activated amino acid, and the reactions are allowed to proceed to yield a plurality of immobilized amino acids, one in each reaction vessel. The aliquots of derivatized beads are then washed, "pooled" (i.e., recombined), and the pool of beads is again divided, with each aliquot being placed in a separate reaction vessel. Another activated amino acid is then added to each aliquot of beads. The cycle of synthesis is repeated until a desired peptide length is obtained. The amino acid residues added at each synthesis cycle can be randomly selected; alternatively, amino acids can be selected to provide a "biased" library, e.g., a library in which certain portions of the inhibitor are selected non-randomly, e.g., to provide an inhibitor having known structural similarity or homology to a known peptide capable of interacting with an antibody, e.g., the an anti-idiotypic antibody antigen binding site. It will be appreciated that a wide variety of peptidic, peptidomimetic, or non-peptidic compounds can be readily generated in this way.

The "split-pool" strategy results in a library of peptides, e.g., inhibitors, which can be used to prepare a library of test compounds of the invention. In another illustrative synthesis, a "diversomer library" is created by the method of Hobbs DeWitt et al. (Proc. Natl. Acad. Sci. U.S.A. 90:6909 (1993)). Other synthesis methods, including the "tea-bag" technique of Houghten (see, e.g., Houghten et al., Nature 354:84-86 (1991)) can also be used to synthesize libraries of compounds according to the subject invention.

Libraries of compounds can be screened to determine whether any members of the library have a desired activity, and, if so, to identify the active species. Methods of screening combinatorial libraries have been described (see, e.g., Gordon *et al., J Med. Chem., supra*). Soluble compound libraries can be screened by affinity chromatography with an appropriate receptor to isolate ligands for the receptor, followed by identification of the isolated ligands by conventional techniques (e.g., mass spectrometry, NMR, and the like). Immobilized compounds can be screened by contacting the compounds with a soluble receptor; preferably, the soluble receptor is conjugated to a label (e.g., fluorophores, colorimetric enzymes, radioisotopes, luminescent compounds, and the like) that can be detected to indicate ligand binding. Alternatively, immobilized compounds can be selectively released and allowed to diffuse through a membrane to interact with a receptor. Exemplary assays useful for screening the libraries of the invention are described below.

In one embodiment, compounds of the invention can be screened for the ability to interact with a pathogenic immunoglobulin by assaying the activity of each compound to bind directly to the immunoglobulin or to inhibit an interaction between the immunoglobulin and an ischemic antigen, e.g., by incubating the test compound with an immunoglobulin and a lysate, e.g., an endothelial cell lysate, e.g., in one well of a multiwell plate, such as a standard 96-well microtiter plate. In this embodiment, the activity of each individual compound can be determined. A well or wells having no test compound can be used as a control. After incubation, the activity of each test compound can be determined by assaying each well. Thus, the activities of a plurality of test compounds can be determined in parallel.

### Protein or Peptide Inhibitors

Protein or peptide inhibitors can be identified using, e.g., the combinatorial systems described above. Alternatively, fragments or peptides of any of the pathogenic immunoglobulin, a target antigen or a component of the complement pathway can be used to block interactions among these proteins.

Also within the scope of the invention are variants of the fragments or peptides of any of the pathogenic immunoglobulin, a target antigen or a component of the complement pathway that include "non-essential" amino acid substitutions. Non-essential amino acid substitutions refer to alterations from the wild-type sequence that can be made without abolishing or more preferably, without substantially altering a biological activity, whereas an "essential" amino acid residue results in such a change.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a pathogenic immunoglobulin can be preferably replaced with another amino acid residue from the same side chain family. Alternatively, in another embodiment, mutations can be introduced randomly along all or part of a pathogenic immunoglobulin coding sequence, such as by saturation mutagenesis, and the resultant mutants can be screened for biological activity.

In nother aspect, the invention also features a modified pathogenic immunoglobulin, e.g., which functions as an agonist (mimetics) or as an antagonist. Preferably the modified pathogenic immunoglobulin functions as an antagonist of complement activation. Variants of the pathogenic immunoglobulin can be generated by mutagenesis, e.g., discrete point mutation, the insertion or deletion of sequences or the truncation of a pathogenic immunoglobulin. An agonist of the pathogenic immunoglobulin can retain substantially the same, or a subset, of the biological activities of the naturally occurring form of the protein. An antagonist of a pathogenic immunoglobulin can inhibit one or more of the activities of the naturally occurring form of the pathogenic immunoglobulin by, for example, being capable of binding to an ischemic specific antigen, but incapable of activating a complement pathway. Thus, specific biological effects can be elicited by treatment with a variant of limited function.

In one embodiment, the site within the pathogenic immunoglobulin (e.g., a pathogenic IgM) that binds C1q can be mutated such that it is no longer capable of binding C1q. For example, the C_{H}2 domain of an IgG and the C_{H}4 domain of an IgM, which are known to contain binding sites for C1q, can be mutated (see WO 94/29351). For example, the carboxyl terminal half of the C_{H}2 domain of an IgG (residues 231 to 239, preferably within 234 to 239), which appear to mediate C1q binding and subsequent complement activation, can be mutated. As another example, Wright et al. have demonstrated that a single nucleotide change in the IgM constant region domain renders the antibody defective in initiating complement-dependent cytolysis. The single nucleotide change results in the encoding of a serine residue, rather than the normal proline residue, at amino acid position 436 in the third constant domain (Wright et al. 1988, J. Biol. Chem. 263: 11221). The amino acid substitutions that can be made to antibodies in order to alter complement binding or activity are well known in the art (see for example, Wright et al. 1988, J. Biol. Chem. 263: 11221; Shulman et al. (1986), Proc. Natl. Acad Sci. USA 83: 7678-7682; Arya et al., (1994) J. Immunol. 253: 1206-1212; Poon et al., (1995) J. Biol Chem. 270: 8571-8577, the contents of all of which are hereby incorporated by reference). Accordingly, in an embodiment, the antibodies of the present invention have a mutation that alters complement binding or activity. Antibodies in which amino acids have been added, deleted, or substituted are referred to herein as modified antibodies or altered antibodies. As will be appreciated by the skilled artisan, the methods used for causing such changes in nucleotide or amino acid sequence will vary depending upon the desired results.

Variants of a pathogenic immunoglobulin can be identified by screening combinatorial libraries of mutants, e.g., truncation mutants, of a pathogenic immunoglobulin for agonist or antagonist activity.

Libraries of fragments e.g., N terminal, C terminal, or internal fragments, of a pathogenic immunoglobulin coding sequence can be used to generate a variegated population of fragments for screening and subsequent selection of variants of this protein. Variants in which a cysteine residue is added or deleted or in which a residue that is glycosylated is added or deleted are particularly preferred.

Methods for screening gene products of combinatorial libraries made by point mutations or truncation, and for screening cDNA libraries for gene products having a selected property. Recursive ensemble mutagenesis (REM), a technique which enhances the frequency of functional mutants in the libraries, can be used in combination with the screening assays to identify variants (Arkin and Yourvan (1992) Proc. Natl. Acad. Sci. USA 89:7811-7815; Delgrave et al. (1993) Protein Engineering 6(3):327-331).

Cell based assays can be exploited to analyze a variegated library. For example, a library of expression vectors can be transfected into a cell line, e.g., a cell line, which ordinarily responds to the protein_in a substrate-dependent manner. Plasmid DNA can then be recovered from the cells which score for inhibition, or alternatively, potentiation of signaling by the pathogenic immunoglobulin-substrate, and the individual clones further characterized.

In another aspect, the invention features a method of making a pathogenic immunoglobulin, e.g., a pathogenic immunoglobulin having a non-wild type activity, e.g., an antagonist, agonist, or super agonist of a naturally occurring pathogenic immunoglobulin. The method includes: altering the sequence of a pathogenic immunoglobulin, e.g., by substitution or deletion of one or more residues of a non-conserved region, a domain or residue disclosed herein, and testing the altered polypeptide for the desired activity.

In another aspect, the invention features a method of making a fragment or analog of a pathogenic immunoglobulin having an altered biological activity of a naturally occurring pathogenic immunoglobulin. The method includes: altering the sequence, e.g., by substitution or deletion of one or more residues, of a pathogenic immunoglobulin, e.g., altering the sequence of a non-conserved region, or a domain or residue described herein, and testing the altered polypeptide for the desired activity.

### Production of Immunoglobulins

In other embodiments, the inhibitors can be an antibody or a fragment thereof, e.g., an anti-idiotypic antibody or an antigen binding portion thereof.

The term "monoclonal antibody" (mAb or mAbs) as used herein refers to an antibody molecule of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

Methods of producing antibodies are well known in the art. For example, a monoclonal antibody against a target (e.g., a pathogenic immunoglobulin or an ischemia specific antigen on a cell) can be produced by a variety of techniques, including conventional monoclonal antibody methodology e.g., the standard somatic cell hybridization technique of Kohler and Milstein, Nature 256: 495 (1975). Although somatic cell hybridization procedures are preferred, in principle, other techniques for producing monoclonal antibody can be employed e.g., viral or oncogenic transformation ofB lymphocytes. The preferred animal system for preparing hybridomas is the murine system. Hybridoma production in the mouse is a very well-established procedure, Immunization protocols and techniques for isolation of immunized splenocytes for fusion are known in the art. Fusion partners (e.g., murine myeloma cells) and fusion procedures are also known.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918, Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

In one embodiment, hybridomas can be generated from human CD5+, B-1 cells. Alternatively, "humanized" murine hybridomas can be used that recognize cross-reactive "ischemic antigen".

Monoclonal antibodies can also be generated by other methods known to those skilled in the art of recombinant DNA technology. An alternative method, referred to as the "combinatorial antibody display" method, has been developed to identify and isolate antibody fragments having a particular antigen specificity, and can be utilized to produce monoclonal antibodies (for descriptions of combinatorial antibody display see e.g., Sastry et al. 1989 PNAS 86:5728; Huse et al. 1989 Science 246:1275; and Orlandi et al. 1989 PNAS 86:3833). After immunizing an animal with an immunogen as described above, the antibody repertoire of the resulting B-cell pool is cloned. Methods are generally known for obtaining the DNA sequence of the variable regions of a diverse population of immunoglobulin molecules by using a mixture of oligomer primers and PCR. For instance, mixed oligonucleotide primers corresponding to the 5' leader (signal peptide) sequences and/or framework 1 (FR1) sequences, as well as primer to a conserved 3' constant region primer can be used for PCR amplification of the heavy and light chain variable regions from a number of murine antibodies (Larrick et al., 1991, Biotechniques 11:152-156). A similar strategy can also been used to amplify human heavy and light chain variable regions from human antibodies (Larrick et al., 1991, Methods: Companion to Methods in Enzymology 2:106-110).

In an illustrative embodiment, RNA is isolated from B lymphocytes, for example, peripheral blood cells, bone marrow, or spleen preparations, using standard protocols (e.g., U.S. Patent No. 4,683,202; Orlandi, et al. PNAS (1989) 86:3833-3837; Sastry et al., PNAS (1989) 86:5728-5732; and Huse et al. (1989) Science 246:1275-1281.) First-strand cDNA is synthesized using primers specific for the constant region of the heavy chain(s) and each of the κ and λ light chains, as well as primers for the signal sequence. Using variable region PCR primers, the variable regions of both heavy and light chains are amplified, each alone or in combinantion, and ligated into appropriate vectors for further manipulation in generating the display packages. Oligonucleotide primers useful in amplification protocols may be unique or degenerate or incorporate inosine at degenerate positions. Restriction endonuclease recognition sequences may also be incorporated into the primers to allow for the cloning of the amplified fragment into a vector in a predetermined reading frame for expression.

The V-gene library cloned from the immunization-derived antibody repertoire can be expressed by a population of display packages, preferably derived from filamentous phage, to form an antibody display library. Ideally, the display package comprises a system that allows the sampling of very large variegated antibody display libraries, rapid sorting after each affinity separation round, and easy isolation of the antibody gene from purified display packages. In addition to commercially available kits for generating phage display libraries (e.g., the Pharmacia Recombinant Phage Antibody System, catalog no. 27-9400-01; and the Stratagene *SurfZAP*^{™} phage display kit, catalog no. 240612), examples of methods and reagents particularly amenable for use in generating a variegated antibody display library can be found in, for example, Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982.

In certain embodiments, the V region domains of heavy and light chains can be expressed on the same polypeptide, joined by a flexible linker to form a single-chain Fv fragment, and the scFV gene subsequently cloned into the desired expression vector or phage genome. As generally described in McCafferty et al., Nature (1990) 348:552-554, complete V_{H} and V_{L} domains of an antibody, joined by a flexible (Gly₄-Ser)₃ linker can be used to produce a single chain antibody which can render the display package separable based on antigen affinity. Isolated scFV antibodies immunoreactive with the antigen can subsequently be formulated into a pharmaceutical preparation for use in the subject method.

Once displayed on the surface of a display package (e.g., filamentous phage), the antibody library is screened with the target antigen, or peptide fragment thereof, to identify and isolate packages that express an antibody having specificity for the target antigen. Nucleic acid encoding the selected antibody can be recovered from the display package (e.g., from the phage genome) and subcloned into other expression vectors by standard recombinant DNA techniques.

Specific antibody molecules with high affinities for a surface protein can be made according to methods known to those in the art, e.g, methods involving screening of libraries (Ladner, R.C., et al., U.S. Patent 5,233,409; Ladner, R.C., *et al*., C.T.S. Patent 5,403,484): Further, the methods of these libraries can be used in screens to obtain binding determinants that are mimetics of the structural determinants of antibodies.

In particular, the Fv binding surface of a particular antibody molecule interacts with its target ligand according to principles of protein-protein interactions, hence sequence data for V_{H} and V_{L} (the latter of which may be of the κ or λ chain type) is the basis for protein engineering techniques known to those with skill in the art. Details of the protein surface that comprises the binding determinants can be obtained from antibody sequence information, by a modeling procedure using previously determined three-dimensional structures from other antibodies obtained from NMR studies or crytallographic data. See for example Bajorath, J. and S. Sheriff, 1996, Proteins: Struct., Funct., and Genet. 24 (2), 152-157; Webster, D.M. and A, R. Rees, 1995, "Molecular modeling of antibody-combining sites,"in S. Paul, Ed., Methods in Molecular Biol. 51, Antibody Engineering Protocols, Humana Press, Totowa, NJ, pp 17-49; and Johnson, G., Wu, T.T. and E.A. Kabat, 1995, "Seqhunt: A program to screen aligned nucleotide and amino acid sequences, in Methods in Molecular Biol. 51, op. cit., pp 1-15.

In one embodiment, a variegated peptide library is expressed by a population of display packages to form a peptide display library. Ideally, the display package comprises a system that allows the sampling of very large variegated peptide display libraries, rapid sorting after each affinity separation round, and easy isolation of the peptide-encoding gene from purified display packages. Peptide display libraries can be in, e.g., prokaryotic organisms and viruses, which can be amplified quickly, are relatively easy to manipulate, and which allows the creation of large number of clones. Preferred display packages include, for example, vegetative bacterial cells, bacterial spores, and most preferably, bacterial viruses (especially DNA viruses). However, the present invention also contemplates the use of eukaryotic cells, including yeast and their spores, as potential display packages. Phage display libraries are described above.

Other techniques include affinity chromatography with an appropriate "receptor", e.g., a target antigen, followed by identification of the isolated binding agents or ligands by conventional techniques (e.g., mass spectrometry and NMR). Preferably, the soluble receptor is conjugated to a label (e.g., fluorophores, colorimetric enzymes, radioisotopes, or luminescent compounds) that can be detected to indicate ligand binding.
Alternatively, immobilized compounds can be selectively released and allowed to diffuse through a membrane to interact with a receptor.

Combinatorial libraries of compounds can also be synthesized with "tags" to encode the identity of each member of the library (see, e.g., W.C, Still *et al*., International Application WO 94/08051). In general, this method features the use of inert but readily detectable tags that are attached to the solid support or to the compounds. When an active compound is detected, the identity of the compound is determined by identification of the unique accompanying tag. This tagging method permits the synthesis of large libraries of compounds which can be identified at very low levels among the total set of all compounds in the library.

The term "modified antibody" is intended to include antibodies, such as monoclonal antibodies, chimeric antibodies, and humanized antibodies which have been modified by, e.g., deleting, adding, or substituting portions of the antibody. For example, an antibody can be modified by deleting the hinge region, thus generating a monovalent antibody. An antibody of the present invention can be one in which the variable region, or a portion thereof, e.g., the complementarity determining regions (CDR or CDRs), are generated in a non-human organism, e.g., a rat or mouse. Chimeric, CDR-grafted, and humanized antibodies are within the invention. Antibodies generated in a non-human organism, e.g., a rat or mouse, and then modified, e.g., in the variable framework or constant region, to decrease antigenicity in a human are within the invention. Any modification is within the scope of the invention so long as the antibody has at least one antigen binding portion.

Chimeric antibodies (e.g. mouse-human monoclonal antibodies) can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted, (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

A chimeric antibody can be further humanized by replacing sequences of the Fv variable region which are not directly involved in antigen binding with equivalent sequences from human Fv variable regions. General methods for generating humanized antibodies are provided by Morrison, S. L., 1985, Science 229:1202-1207 by Oi et al., 1986, BioTechniques 4:214, and by Queen et al. US 5,585,089, US 5,693,761 and US 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from 7E3, an anti-GPII_{b}IIIₐ antibody producing hybridoma. The recombinant DNA encoding the chimeric antibody, or fragment thereof, can then be cloned into an appropriate expression vector. Suitable humanized antibodies can alternatively be produced by CDR substitution. U.S. Patent 5,225,539; Jones et al. 1986 Nature 321:552-525; Verhoeyan et al. 1988 Science 239:1534; and Beidler et al. 1988 J. Immunol. 141:4053-4060.

Humanized or CDR-grafted antibodies can be produced by CDR-grafting or CDR substitution, wherein one, two, or all CDR's of an immunoglobulin chain can be replaced. See e.g., U.S. Patent 5,225,539; Jones et al. 1986 Nature 321:552-525; Verhoeyan et al. 1988 Science 239:1534; Beidler et al. 1988 J. Immunol. 141:4053-4060; Winter US 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US 5,225,539), the contents of which is expressly incorporated by reference.

A humanized or CDR-grafted antibody will have at least one or two but generally all recipient CDR's (of heavy and/or light immunoglobulin chains) replaced with a donor CDR. Preferably, the donor will be a rodent antibody, e.g., a rat or mouse antibody, and the recipient will be a human framework or a human consensus framework. Typically, the immunoglobulin providing the CDR's is called the "donor" and the immunoglobulin providing the framework is called the "acceptor." In one embodiment, the donor immunoglobulin is a non-human (e.g., rodent). The acceptor framework can be a naturally-occurring (e.g., a human) framework or a consensus framework, or a sequence about 85% or higher, preferably 90%, 95%, 99% or higher identical thereto.

All of the CDRs of a particular antibody may be replaced with at least a portion of a non-human CDR or only some of the CDRs may be replaced with non-human CDRs. It is only necessary to replace the number of CDR's required for binding of the humanized antibody to the Fc receptor.

Also within the scope of the invention are chimeric and humanized antibodies in which specific amino acids have been substituted, deleted or added. In particular, preferred humanized antibodies have amino acid substitutions in the framework region, such as to improve binding to the antigen. For example, a humanized antibody will have framework residues identical to the donor framework residue or to another amino acid other than the recipient framework residue. As another example, in a humanized antibody having mouse CDRs, amino acids located in the human framework region can be replaced with the amino acids located at the corresponding positions in the mouse antibody. Such substitutions are known to improve binding of humanized antibodies to the antigen in some instances.

As described above, the term "antigen-binding portion" or "antibody portion" or "fragment", as used herein, refers to one or more fragments of an antibody that retain the ability to specifically bind to an antigen (*e*.*g*. a target antigen, e.g., an ischemic antigen). Antibody fragments of the invention are obtained using conventional procedures known to those with skill in the art. For example, digestion of an antibody with pepsin yields F(ab')₂ fragments and multiple small fragments. Mercaptoethanol reduction of an antibody yields individual heavy and light chains. Digestion of an antibody with papain yields individual Fab fragments and the Fc fragment, Antibody fragments are screened for utility in the same manner as are intact antibodies, as described above.

The nucleotide sequence of the 22A5 IgM heavy chain variable region is shown in Figure 4A (SEQ ID NO:1), and the amino acid sequence of the 22A5 IgM heavy chain variable region is shown in Figure 5A (SEQ ID NO:2). The CDR1 domain of the heavy chain variable region corresponds to amino acids about 30 to 34 of SEQ ID NO:2 (SEQ ID NO:4) and encompasses nucleotides about 91-105 of SEQ ID NO:1 (SEQ ID NO:3) and the CDR2 domain of the heavy chain variable region corresponds to amino acids 49 to 65 of SEQ ID NO:2 (SEQ ID NO:6) and encompasses nucleotides 1.48-198 of SEQ ID NO:1 (SEQ ID NO:5).

The nucleotide sequence of the 22A5 IgM light chain variable region is shown in Figure 4B (SEQ ID NO:7), and the amino acid sequence of the 22A5 lgM light chain variable region is shown in Figure 5B (SEQ ID NO:8). The CDR1 domain of the light chain variable region corresponds to amino acids about 23 to 33 of SEQ ID NO:8 (SEQ ID NO:10) and encompasses nucleotides about 71-103 of SEQ ID NO:7 (SEQ ID NO.9) and the CDR2 domain of the light chain variable region corresponds to about amino acids 49 to 55 of SEQ ID NO:8 (SEQ ID NO:12) and encompasses nucleotides about 149 to 169 of SEQ ID NO:7 (SEQ ID NO:11).

It will be appreciated by the skilled artisan that nucleotide sequences encoding the antibodies of the present invention, or fragments thereof, (*e*.*g*., a CDR domain, such as a CDR2 domain, or fragments), can be derived from the nucleotide and amino acid sequences described in the present application using the genetic code and standard molecular biology techniques. Further, the skilled artisan will appreciate that due to the degeneracy of the genetic code, other nucleotide sequences can encode the amino acid sequences listed herein.

The nucleic acid compositions of the present invention, while often in a native sequence (except for modified restriction sites and the like), from either cDNA, genomic or mixtures may be mutated, thereof in accordance with standard techniques to provide gene sequences. For coding sequences, these mutations, may affect the amino acid sequence as desired. In particular, nucleotide sequences substantially identical to or derived from native V, D, J, constant, switches and other such sequences described herein are contemplated (where "derived" indicates that a sequence is identical or modified from another sequence).

In one embodiment, an isolated nucleic acid comprises a 22A5 IgM heavy chain variable region nucleotide sequence having a nucleotide sequence as shown in Figure 4A (SEQ ID NO:1), or a sequence at least 96%, 97%, 98%, 99% or higher identical thereto. In another embodiment, the isolated nucleic acid encodes a 22A5 IgM light chain variable region nucleotide sequence having a nucleotide sequence as shown in Figure 4B (SEQ ID NO:7), or a sequence at least 96%, 97%, 98%, 99% or higher identical thereto.

In another embodiment, the invention provides an isolated nucleic acid encoding a heavy chain CDR1 domain comprising the amino acid sequence of SEQ ID NO: 4, or a fragment or modified form thereof. This nucleic acid can encode only the CDR1 region or can encode an entire antibody heavy chain variable region. For example, the nucleic acid can encode a heavy chain variable region having a CDR2 domain comprising the amino acid sequence of SEQ ID NO:6. In yet another embodiment, the invention provides an isolated nucleic acid encoding a heavy chain CDR2 domain comprising the amino acid sequence of SEQ ID NO:6, or a fragment or modified form thereof. This nucleic acid can encode only the CDR2 region or can encode an entire antibody heavy chain variable region. For example, the nucleic acid can encode a light chain variable region having a CDR1 domain comprising the amino acid sequence of SEQ ID NO:4.

In still another embodiment, the invention provides an isolated nucleic acid encoding a light chain CDR1 domain comprising the amino acid sequence of SEQ ID NO: 10, or a fragment or modified form thereof This nucleic acid can encode only the CDR1 region or can encode an entire antibody light chain variable region. For example, the nucleic acid can encode a light chain variable region having a CDR2 domain comprising the amino acid sequence of SEQ ID NO:12. In yet another embodiment, the invention provides an isolated nucleic acid encoding a light chain CDR2 domain comprising the amino acid sequence of SEQ ID NO:12, or a fragment or modified form thereof. This nucleic acid can encode only the CDR2 region or can encode an entire antibody light chain variable region. For example, the nucleic acid can encode a light chain variable region having a CDR1 domain comprising the amino acid sequence of SEQ ID NO:10.

### Pharmaceutical Compositions and Administration

The inhibitors of the invention can be incorporated into pharmaceutical compositions suitable for administration to a subject. Typically, the pharmaceutical composition comprises an inhibitor of the invention (e.g. a modified pathogenic immunoglobulin) and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody or antibody portion,

A pharmaceutical composition is formulated to be compatible with its intended route of administration. Examples of routes of administration can include, for example, parenteral, e.g., intravenous, intradermal, subcutaneous, oral (e.g., inhalation), transdermal (topical), transmucosal, and rectal administration. Thus the compositions of this invention may be in a variety of forms. These include, for example, liquid, semisolid and solid dosage forms, such as liquid solutions (*e*.*g*., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. A preferred mode of administration is parenteral (*e*.*g*., intravenous, subcutaneous, intraperitoneal, intramuscular). In a preferred embodiment, the antibody is administered by intravenous infusion or injection. In another preferred embodiment, the antibody is administered by intramuscular or subcutaneous injection.

Solutions or suspensions used for parenteral, e.g. intradermal or subcutaneous, application can, for example, include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Therapeutic compositions typically must be sterile and stable under the conditions of manufacture and storage, The composition can be formulated as a solution, microemulsion, dispersion, liposome, or other ordered structure suitable to high drug concentration. Sterile injectable solutions can be prepared by incorporating the active compound (*i*.*e*., antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants.

The inhibitors of the present invention can be administered by a variety of methods known in the art, although for many therapeutic applications, the preferred route/mode of administration is intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e*.*g*., Sustained and Controlled Release Drug Delivery Systems, J.R. Robinson, ed., Marcel Dekker, Inc., New York, 1978.

In certain embodiments, an inhibitor of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation. For administration by inhalation, the compounds are delivered in the form of an aerosol spray from pressured container or dispenser which contains a suitable propellant, e.g., a gas such as carbon dioxide, or a nebulizer.

The inhibitor of the invention can be prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc.
Liposomal suspensions can also be used as pharmaceutically acceptable carriers. These can be prepared according to methods known to those skilled in the art, for example, as described in U.S. Patent No. 4,522,811.

The pharmaceutical compositions can be included in a container, pack, or dispenser together with instructions for administration.

### EXAMPLES

### Example 1: Mechanism of Ischemia-Reperfusion Injury

This Example shows that mice deficient in the complement system were resistant to ischemia-reperfusion injury.

To examine the mechanism of ischemia-reperfusion injury, mice deficient in complement C3 were treated in the hindlimb model. The C3-/- mice were partially protected from injury based on an approximate 50% reduction in permeability index (see Weiser et al. (1996) J. Exp. Med. 1857-1864). Thus, complement C3 is essential for induction of full injury in this murine model.

The experiments in Weiser et al. did not identify how complement was activated.
The serum complement system can be activated by at least three distinct pathways, classical, lectin or alternative. Knowing which pathway is involved, is important as it suggests a mechanism for injury. For example, the classical pathway is activated very efficiently by IgM and IgG isotypes of immunoglobulin or by the serum recognition protein C-reactive protein. Whereas, the lectin pathway is activated following recognition of specific carbohydrates such as mannan by mannan binding lectin (MBL) (Epstein et al., (1996) Immunol 8, 29-35). In both pathways, complement C4 is required in forming an enzyme complex with C2 that catalyzes cleavage of the central component C3. By contrast, the alternative pathway activates spontaneously leading to conversion of C3 to its active form (C3b) and attachment to foreign-or self-tissues. The pathway is tightly regulated as all host cells express inhibitors of amplification of the complement pathway by inactivating, or displacing the C3 convertase (Muller-Eberhard, H.J., (1988) Ann. Rev. Biochem. 57. 321-347). One approach for determining the pathway involved is use of mice deficient in C4, i.e. cannot form C3 convertase via classical or lectin pathways. Comparison of mice deficient in either C3 or C4 with wild type (WT) controls in the hindlimb model, revealed that C4 was also required for induction of full injury (Weiser et al. *supra*). This finding was important as it suggested that antibody or MBL might be involved.

### Example 2: Natural IgM Mediates Ischemia Reperfusion (I/R) Injury.

This Example shows that mice deficient in immunoglobulin were resistant to ischemia-reperfusion injury.

To determine if antibody was involved in mediating I/R injury, mice totally deficient in immunoglobulin, RAG2-/- (recombinase activating gene-2 deficient) were characterized along with the complement deficient animals in the intestinal model. Significantly, the RAG-2-/- mice were protected to a similar level as observed in the complement deficient animals (Weiser et al. *supra*). Since the RAG2-/- animals are also missing mature lymphocytes, it was important to determine that the pathogenic effect was antibody dependent (Shinkai et al. (1992) Cell 68, 855-867). To confirm that injury was mediated by serum antibody, the deficient animals were reconstituted with either normal mouse sera (Weiser et al. *supra*) or purified IgM (Williams et al. (1999) J. Appl Physiol 86; 938-42). In both cases, the reconstituted RA.G-2-/- mice were no longer protected and injury was restored. In the latter experiments, a model of intestinal injury was used as in this model, injury is thought to be mediated primarily by complement.

The interpretation of these results is that during the period of ischemia, neo-antigens are either expressed or exposed on the endothelial cell surface. Circulating IgMs appear to recognize the new determinant, bind and activate classical pathway of complement. While the nature of the antigen is not known, IgM rather than IgG seems to be primarily responsible for activation of complement as reconstitution of deficient mice with pooled IgG did not significantly restore injury in the mice. An alternative hypothesis is that there is another initial event such as the MBL pathway that recognizes the altered endothelial surface, induces low level complement activation which in turn exposes new antigenic sites and the pathway is amplified by binding of IgM.

### Example 3: Pathogenic IgM is a Product of B-1 cells:

Since a major fraction of circulating IgM is thought to represent natural antibody, i.e. product of rearranged germline genes, it is possible that mice bearing deficiencies in the B-1 fraction of lymphocytes might also be protected. B-1 cells have a distinct phenotype from more conventional B-2 cells in that they express low levels of TgD and CD23 and a major fraction express the cell surface protein CD5 (Hardy et al., (1994) Immunol. Rev.: 137, 91; Kantor et al. (1993) Annu. Rev. Immunol. 11, 501-538, 1993. B-1 cells are also distinguished by reduced circulation in mice, limited frequency in the peripheral lymph nodes and spleen and are primarily localized within the peritoneal cavity. To examine a role for B-1 cells as a source of pathogenic IgM, antibody-deficient mice (RAG-2-/-) were reconstituted with 5 X 10⁵ peritoneal B-1 cells and rested approximately 30 days before treatment. Circulating IgM levels reach a near normal range within a month following adoptive transfer. Characterization of the B-1 cell reconstituted mice in the intestinal ischemia model confirmed that B-1 cells were a major source of pathogenic IgM (see Williams et al. (1999) supra). This was an important observation because the repertoire of B-1 cell natural antibody is considerably more limited than would be expected for conventional B-2 cells. Therefore, it is possible that the pathogenic antibody represents a product of the germline.

### Example 4: Cr2-/- Mice Are Protected from Ischemia Reperfusion Injury:

The initial characterization of Cr2-/- knockout mice revealed an approximate 50% reduction in the frequency of B-1a or CD5 + B-1 cells (Ahearn et al. (1996) Immunity 4: 251-262). Although characterization of another strain of Cr2-deficient mice did not identify a similar reduction (Molina et al. (1996) Proc. Natl. Acad. Sci. USA 93, 3357-3361). Whether the difference in frequency of CD5 + cells was due to variation in strain background or environmental differences is not known. Despite the reduced frequency of B-1a cells in the Cr2-/- mice, circulating levels of IgM were within the normal range. These findings suggested that the repertoire of IgM might be different in the Cr2-deficient animals. To test this hypothesis, mice in the intestinal I/R model were characterized. Surprisingly, the Cr2-/- mice were equally protected as the complete-antibody deficient mice (Figure 1). Comparison of survival over a five-day period following treatment in the intestinal model demonstrated a significant increase in mortality of the WT compared to Cr2-deficient animals. Consistent with an increased mortality, a dramatic reduction in injury was observed in tissue sections harvested from treated WT or Cr2-/- deficient mice.

Extensive injury to the mucosal layer of the intestine was observed in WT mice or Cr2-/- mice reconstituted with pooled IgM or B-1 cells, By contrast, tissue sections isolated from treated Cr2-/- mice were similar to that of sham controls. Thus, despite normal circulating levels of IgM, the Cr2-deficient mice were protected from injury. These results not only confirm the importance of B-1 cells as a source of pathogenic antibody but suggest that the complement system is somehow involved in formation or maintenance of the repertoire of natural antibody, For example, complement may be involved in positive selection of B-1 cells,

### Example 5: Identification of Pathogenic IgMs

This Example describes the generation of a specific hybridoma clone from normal B-1 cells and the identification of one clone that produces a pathogenic IgM. The pathogenic IgM was shown to restore injury *in vivo* to antibody-deficient mice.

Studies in mice bearing a deficiency in complement receptors CD21/CD35, revealed that the mice were missing the pathogenic antibody. This finding was unexpected because they have a normal level of IgM in their blood. These findings led to the hypothesis that a special population ofB cells termed B-1 cells are responsible for secreting the pathogenic IgM. For example, engraftment of the receptor deficient mice (Cr2-/-) with B-1 cells from normal mice restored injury, confirming the importance of B-1 cells. To identify the specific antibody or antibodies responsible for injury, a panel of hybridoma clones were constructed from an enriched pool of peritoneal B-1 cells harvested from normal mice. The general approach for preparing hybridomas from enriched fraction of peritoneal cells includes harvesting peritoneal cells from mice treated 7 days earlier with IL-10 and subsequently enriched for CD23⁺ B cells by negative selection with magnetic beads. Enriched B cells are analyzed by FACS following staining with IgM, Mac-1 and CD23 specific Mab. The enriched population is further activated by culturing with LPS for 24 hours. Activated cells are hybridized with fusion partner myeloma cells in the presence of PEG and grown in HAT-selective meium. Hybridomas are screened for IgM secreting clones by ELISA, and positive wells are expanded for purification of IgM.

Twenty-two IgM-secreting hybridoma clones were analyzed by pooling an equal amount of IgM product from each of the clones. Treatment of antibody-deficient mice with the pooled IgM restored injury similar to that seen with pooled IgM from serum. This finding confirmed that the pathogenic IgM was among the twenty-two hybridomas produced. By dividing the pools into two fractions, i.e., 1-11 and 12-22, and treatment mice with the two fractions, the pathogenic antibody was found to fractionate with the pool that included clone # 22. Finally, mice were reconstituted with either clone 17 or 22. Clone 22 restored injury whereas the other clones did not (see Fig. 3A and 3B).

### Example 6: Complement involvement in B-1 cell selection:

Two different models have been proposed to explain the development of B-1 cells. The lineage hypothesis proposes that B-1 cells develop in early fetal life as a distinct population (Kantor et al. (1993) *supra*). Alternatively, B-1 cells develop from the same progenitors as conventional B cells but depending on their environment, i.e. encounter with antigen, they develop into B-1 or retain the B-2 cells phenotype (Wortis, H.H. (1992) Int. Rev. Immunol. 8, 235; Clarke, J. (1998) Exp. Med. 187, 1325-1334). Irrespective of their origin, it is known that B-1 cells are not replenished from adult bone marrow at the same frequency as B-2 cells and that their phenotype is more similar to that of early fetal liver B cells or neonatal bone marrow (BM) cells. Consistent with an early origin, their repertoire tends to be biased towards expression of more proximal VH genes and N-nucleotide addition is limited (Gu et al. (1990) EMBO J 9, 2133; Feeney, J. (1990) Exp. Med. 172, 1377). It seems reasonable that given the reduced replenishment by adult BM stem cells, B-1 cells are self renewed and that antigen stimulation might be important in their renewal, expansion or even initial selection (Hayakawa et al., (1986) Eur. J. Immunol. 16, 1313). Indeed inherent to the conventional model, B-1 cells must be antigen selected.

Evidence in support of a B-cell receptor (BCR) signaling requirement for positive selection of B-1 cells comes from mice bearing mutations that alter BCR signaling. For example, impairment of BCR signaling through CD19 (19, 20), vav (21) or Btk (22) dramatically affects development of B-1 cells. By contrast, loss of negative selection such as in CD22- or SHP-1 deficient mice can lead to an increase in B-1 cell frequency (O'Keefe et al. (1996) Science 274, 798-801; Shultz et al. (1993) Cell 73, 1445). Recent, elegant studies with mice bearing two distinct Ig transgenes, V_{H}12 (B-1 cell phenotype) or V_{H}B1-8 (B-2 cell phenotype) support the view that B-1 cells are positively selected by self-antigens. For example, B cells expressing V_{H}12 either alone or together with B1-8 developed a B-1 cell phenotype. Whereas, few if any B cells were identified that expressed the B1-8 tg only. Thus, these results suggested that encounter of transgenic B cells with self-PtC resulted in expansion of those expressing V_{H}12. Selection of B-1 cells was recently reported by Hardy et al. (1994) Immunol. Rev. 137, 91). In their model, B cells expressing an immunoglobulin transgene specific for Thy 1.1 were selected and expanded in mice expressing the cognate antigen. By contrast, transgene + B-1 cells were not found in mice that expressed the alternative allotype Thy 1.2.

Where does complement fit into B-1 cell development? The overall reduction in B-1a cell frequency and the more specific loss of B-1 cells expressing IgM involved in I/R injury suggests a role for CD21/CD35 in either positive selection or maintenance of B-1a cells. One possible role for complement is that it enhances BCR signaling on encounter with cognate antigen. Biochemical studies and analysis of CD21/CD35 deficient mice demonstrate the importance of co-receptor signaling in activation and survival of conventional B cells (Carroll, M.C., (1998) Ann. Rev. Immunol 16, 545-568; Fearon et al. (1995) Annu. Rev. Immunol 13, 127-149). It is very likely that B-1 cells likewise utilize co-receptor signaling to enhance the BCR signal. For example, bacteria express typical B-1 cell antigens such as phosphoryl choline and it is not unreasonable that coating of bacteria with complement ligand C3d would enhance crosslinking of the co-receptor with the BCR and enhance overall signaling. Thus, antigens expressed at lower concentrations might require complement enhancement in order for the cognate B cell to recognize it and expand or be positively selected. Another role for complement receptors is in localizing antigen on follicular dendritic cells (FDC) within the lymphoid compartment. However, since the major population of B-1 cells occupy the peritoneal tissues it is not clear if they would encounter FDC within lymphoid structures. The actual site or sites in which B-1 cells undergo positive selection are not known. It is possible that they must encounter cognate antigen in early fetal development or in neonatal BM. If this is the case, it might be expected that complement receptors on stromal cells within these compartments bind antigen for presentation to B cells. It is possible that complement receptors could participate in both stages of development. First, they might enhance antigens signaling in initial positive selection. Secondly, as selected B-1 cells are replenished at peripheral sites, complement receptors might again be involved in enhancement of BCR signaling.

Figure 2 is a schematic diagram of the proposed role for complement and complement receptors in positive selection of peritoneal B-1 lymphocytes. The interaction of complement-ligand coated antigens (self- and non-self) results in co-ligation of the CD21/CD19 co-receptor and BCR on the cell surface leading to enhanced signaling and positive selection.

### Equivalents

Those skilled in the art will recognize, or be able to ascertain using no more than routine experimentation, many equivalents to the specific embodiments of the invention described herein. Such equivalents are intended to be encompassed by the following claims.

Further aspects and embodiments of the invention can be understood from the following clauses:
A1. A method for treating or preventing immunoglobulin-mediated reperfusion or ischemic injury, in a subject, comprising: administering to the subject an effective amount of an inhibitor of an interaction between a pathogenic immunoglobulin and an ischemia-specific antigen; thereby reducing the number of the pathogenic immunoglobulins bound to the ischemia-specific antigen, such that the injury is inhibited or reduced.
A2. A method for treating or preventing immunoglobulin-mediated reperfusion or ischemic injury, in a subject, comprising : administering to the subject an effective amount of an inhibitor of an interaction between a pathogenic immunoglobulin and a component of the complement pathway; thereby reducing the number of pathogenic immunoglobulins which activate complement activity, such that the injury is inhibited or reduced.
A3. The method of clause A1 or A2, wherein the pathogenic immunoglobulin is an IgM.
A4. The method of clause A1 or A2, wherein the pathogenic immunoglobulin is a subclass of IgMs.
A5. The method of either clause A1 or A2, wherein the pathogenic immunoglobulin is produced by a subpopulation of B cells.
A6. The method of clause A1, wherein the ischemia-specific antigen is present on the surface of an endothelial cell or parenchymal tissue.
A7. The method of clause A1 or A2, wherein the subject is a mammal.
A8. The method of clause A7, wherein the mammal is a human.
A9. The method of either clause A1 or A2, wherein the reperfusion or ischemic injury results following a naturally occurring episode.
A10. The method of either clause A1 or A2, wherein the reperfusion or ischemic injury occurs during or following a surgical procedure.
A11. The method of clause A10, wherein the surgical procedures is selected from the group consisting of angioplasty, stenting procedure, atherectomy, and bypass surgery.
A12. The method of either clause A1 or A2, wherein the inhibitor is selected from the group consisting of a protein, a peptide, a small organic molecule, an antibody or a fragment thereof, a carbohydrate and a glycoprotein.
A13. The method of either of clauses A1 or A2, wherein the injury occurs in a cardiovascular tissue.
A14. A method for treating or preventing immunoglobulin-mediated reperfusion or ischemic injury, in a subject, comprising : removing from the subject or inactivating a pathogenic immunoglobulin or a B cell producing the pathogenic immunoglobulin, thereby reducing the amount of the pathogenic immunoglobulin or B cells present in the subject.
A15. The method of clause A14, wherein the removing or inactivating step is performed ex vivo.
A16. The method of clause A15, wherein the removing or inactivating step of the pathogenic immunoglobulin is performed by administering to the subject an antiidiotypic antibody.
A17. The method of clause A16, wherein the removing or inactivating step of the B cell is performed by administering to the subject a B cell targeting moiety coupled to a toxin.
A18. The method of clause A14, wherein the subject is a mammal.
A19. The method of clause A18, wherein the mammal is a human.
A20. The method of clause A14, wherein the injury occurs in vivo.
A21. The method of clause A14, wherein the injury occurs in a cardiovascular tissue.
A22. An isolated pathogenic immunoglobulin, or antigen binding portion thereof, having one or more of the following properties: (i) is capable of interacting with an ischemia-specific antigen ; (ii) is capable of fixing complement; or (iii) is produced by a subpopulation of B cells.
A23. The pathogenic immunoglobulin of clause A22, which is an IgM.
A24. A modified pathogenic immunoglobulin having a mutation that alters complement binding or activity.
A25. The modified pathogenic immunoglobulin of clause A24, which is capable of interacting with an ischemia-specific antigen.
A26. The modified pathogenic immunoglobulin of clause A24, which is an IgM.
A27. A method for isolating an ischemia-specific antigen, comprising: providing a pathogenic immunoglobulin; contacting a sample containing the ischemia-specific antigen with the pathogenic immunoglobulin under conditions that allow specific binding of the pathogenic immunoglobulin to the sample ; detecting any changes in the level of binding of the pathogenic antibody to the sample relative to a control, wherein a change in the level of binding of the pathogenic immunoglobulin in the presence of the sample relative to that detected in the control is indicative of specific binding; and isolating the ischemia-specific antigen from the sample.
A28. The method of clause A27, wherein the sample is enriched in the ischemia specific antigen.
A29. The method of clause A27, wherein the sample is obtained from a subject with reperfusion or ischemic injury.
A30 The method of clause A27, wherein the sample is a biochemical isolate.
A31 The method of clause A27, wherein the sample is an expression library.
A32. A method for identifying an inhibitor of an interaction between a pathogenic immunoglobulin and an ischemia-specific antigen from a plurality of test compounds, comprising : providing a reaction mixture which includes the pathogenic immunoglobulin and the ischemia-specific antigen under conditions that allow binding of the pathogenic immunoglobulin and the ischemia-specific antigen to occur; contacting the pathogenic immunoglobulin and the ischemia-specific antigen with one or more test compounds, and detecting any changes in binding of the pathogenic immunoglobulin and the ischemia-specific antigen in the presence of a given test compound relative to that detected in the absence of the test compound, wherein a change in the level of binding between the pathogenic immunoglobulin and the ischemia-specific antigen in the presence of the test compound relative to that detected in the absence of the test compound indicates that the test compound is an inhibitor of the interaction between the pathogenic immunoglobulin and the ischemia-specific antigen.
A33. A method for identifying an inhibitor of an interaction between a pathogenic immunoglobulin and component of the complement pathway from a plurality of test compounds, comprising : providing a reaction mixture which includes the pathogenic immunoglobulin and the component of the complement pathway under conditions that allow binding of the pathogenic immunoglobulin and the component of the complement pathway to occur; contacting the pathogenic immunoglobulin and the component of the complement pathway with one or more test compounds, and detecting any changes in binding of the pathogenic immunoglobulin and the component of the complement pathway in the presence of a given test compound relative to that detected in the absence of the test compound, wherein a change in the level of binding between the pathogenic immunoglobulin and the component of the complement pathway in the presence of the test compound relative to that detected in the absence of the test compound indicates that the test compound is an inhibitor of the interaction between the pathogenic immunoglobulin and the component of the complement pathway.
A34. The method of either clause A32 or A33, wherein the pathogenic immunoglobulin is a pathogenic IgM.
A35. The method of either clause A32 or A33, wherein the ischemia-specific antigen is obtained from an endothelial tissue or an endothelial lysate.
A36. The method of either clause A32 or A33, which is performed in vitro.
A37. The method of either clause A32 or A33, wherein the pathogenic immunoglobulin is labeled with a detectable signal.
A38. The method of either clause A32 or A33, wherein the ischemia-specific antigen is labeled with a detectable signal.
A39. The method of either clause A32 or A33, further comprising repeating at least one step.
A40. The method of either clause A32 or A33, wherein the plurality of test compounds, comprises at least 10, 102,10³,10⁴,10⁵,10⁶,10⁷, or 10⁸ compounds.
A41. The method of either clause A32 or A33, wherein the test compound is a peptide or a small organic molecule.
A42. An inhibitor of an interaction between a pathogenic immunoglobulin and an ischemia-specific antigen identified by the method of clause A32.
A43. An inhibitor of an interaction between a pathogenic immunoglobulin and a component of the complement pathway identified by the method of clause A33.
A44. A method for treating or preventing immunoglobulin-mediated reperfusion or ischemic injury, comprising : administering to a subject an agent having a property of inhibiting an interaction between a pathogenic immunoglobulin and an ischemia-specific antigen as determined by the method of ; providing a reaction mixture which includes the pathogenic immunoglobulin and the ischemia-specific antigen under conditions that allow the binding of the pathogenic immunoglobulin and the ischemia-specific antigen to occur; contacting the pathogenic immunoglobulin and the ischemia-specific antigen with the agent, and detecting any changes in binding of the pathogenic immunoglobulin and the ischemia-specific antigen in the presence of the agent relative to that detected in the absence of the agent, wherein a change in the level of binding between the pathogenic immunoglobulin and the ischemia-specific antigen in the presence of the agent relative to that detected in the absence of the agent indicates that the agent is an inhibitor of the interaction between a pathogenic immunoglobulin and the ischemia-specific antigen.
A45. A method for treating or preventing immunoglobulin-mediated reperfusion or ischemic injury, comprising: administering to a subject an agent having a property of inhibiting an interaction between a pathogenic immunoglobulin and a component of the complement pathway as determined by the method of : providing a reaction mixture which includes the pathogenic immunoglobulin and the component of the complement pathway under conditions that allow the binding of the pathogenic immunoglobulin and the component of the complement pathway to occur ; contacting the pathogenic immunoglobulin and the component of the complement pathway with the agent, and detecting any changes in binding of the pathogenic immunoglobulin and the component of the complement pathway in the presence of the agent relative to that detected in the absence of the agent, wherein a change in the level of binding between the pathogenic immunoglobulin and the component of the complement pathway in the presence of the agent relative to that detected in the absence of the agent indicates that the agent is an inhibitor of the interaction between a pathogenic immunoglobulin and the component of the complement pathway.
A46. A method for treating or preventing immunoglobulin-mediated reperfusion or ischemic injury, comprising : administering to a subject an agent, wherein said agent is capable of inhibiting an interaction between a pathogenic immunoglobulin and an ischemia-specific antigen when tested in the following assay: providing a reaction mixture which includes the pathogenic immunoglobulin and the ischemia-specific antigen under conditions that allow the binding of the pathogenic immunoglobulin and the ischemia-specific antigen to occur; contacting the pathogenic immunoglobulin and the ischemia-specific antigen with the agent, and detecting any changes in binding of the pathogenic immunoglobulin and the ischemia-specific antigen in the presence of the agent relative to that detected in the absence of the agent, wherein an inhibition in the level of binding between the pathogenic immunoglobulin and the ischemia-specific antigen in the presence of the agent relative to that detected in the absence of the agent indicates that the agent is an inhibitor of the interaction between a pathogenic immunoglobulin and the ischemia-specific antigen.
A47. A method for treating or preventing immunoglobulin-mediated reperfusion or ischemic injury, comprising: administering to a subject an agent, wherein said agent is capable of inhibiting an interaction between a pathogenic immunoglobulin and a component of the complement pathway when tested in the following assay: providing a reaction mixture which includes the pathogenic immunoglobulin and the component of the complement pathway under conditions that allow the binding of the pathogenic immunoglobulin and the component of the complement pathway to occur; contacting the pathogenic immunoglobulin and the component of the complement pathway with the agent, and detecting any changes in binding of the pathogenic immunoglobulin and the component of the complement pathway in the presence of the agent relative to that detected in the absence of the agent, wherein an inhibition in the level of binding between the pathogenic immunoglobulin and the component of the complement pathway in the presence of the agent relative to that detected in the absence of the agent indicates that the agent is an inhibitor of the interaction between a pathogenic immunoglobulin and the component of the complement pathway.
A48. The isolated pathogenic immunoglobulin of clause A22, wherein the immunoglobulin is produced by B-1 cells.
A49. The isolated pathogenic antibody of clause A22, which is produced by the hybridoma which will be deposited with the ATCC, having Accession Number .
A50. The isolated pathogenic immunoglobulin of clause A22, which has a light chain variable region comprising the amino acid sequence of SEQ IID NO : 8.
A51. The isolated pathogenic immunoglobulin of clause A22, which has a heavy chain variable region comprising the amino acid sequence of SEQ ID NO : 2.
A52. The isolated pathogenic immunoglobulin of clause A22, wherein the immunoglobulin has a light chain variable region comprising a CDR1 region comprising the amino acid sequence shown in SEQ ID NO : 10.
A53. The isolated pathogenic immunoglobulin of clause A22, wherein the immunoglobulin has a light chain variable region comprising a CDR2 region comprising the amino acid sequence shown in SEQ ID NO : 12.
A54. The isolated pathogenic immunoglobulin of clause A22, wherein the immunoglobulin has a heavy chain variable region comprising a CDR1 region comprising the amino acid sequence of SEQ ID NO: 4.
A55. The isolated pathogenic antibody of clause A22, wherein the immunoglobulin has a heavy chain variable region comprising a CDR2 region comprising the amino acid sequence shown in SEQ ID NO: 6.
A56. The isolated pathogenic immunoglobulin of clause A22, wherein the immunoglobulin has a light chain variable region comprising the amino acid sequence shown in SEQ ID NO: 8, and a heavy chain variable region comprising the amino acid sequence shown in SEQ ID NO: 2.
A57. The isolated pathogenic immunoglobulin of clause A22, wherein the immunoglobulin is a human immunoglobulin.
A58. The isolated pathogenic antibody of clause A22, wherein the immunoglobulin is a non-human immunoglobulin.
A59. Then isolated pathogenic immunoglobulin of clause of clause A58, wherein the non-human antibody is from a mammal.
A60. The isolated pathogenic immunoglobulin of clause A59, wherein the mammal is selected from the group consisting of cow, goat, mouse, rat, sheep, pig, and rabbit.
A61. The isolated pathogenic immunoglobulin of clause A22, wherein the immunoglobulin is a recombinant antibody.
A62. The isolated pathogenic immunoglobulin of clause A22, wherein the immunoglobulin comprises the heavy chain CDR1 and CDR2 regions shown in SEQ ID NO : 4 and SEQ ID NO : 6, or antigen binding fragments thereof, and the light chain CDR1 and CDR2 regions shown in SEQ ID NO : 10 and SEQ ID NO : 12, or antigen binding fragments thereof.
A63. The isolated pathogenic antibody of clause A62, wherein the immunoglobulin comprises human framework regions.
A64. An isolated nucleic acid molecule selected from the group consisting of a) a nucleic acid molecule comprising a nucleotide sequence which is at least 96% identical to the nucleotide sequence of SEQ ID NO: 1, SEQ ID NO: 3 or SEQ ID NO : 5 ; b) a nucleic acid molecule comprising the nucleotide sequence of SEQ ID NO: 1, SEQ ID NO : 3 or SEQ ID NO : 5 ; and c) a nucleic acid molecule which hybridizes to the nucleotide sequence of SEQ ID NO: 1 under stringent conditions.
A65. An isolated nucleic acid molecule which encodes a polypeptide comprising the amino acid sequence of SEQ ID NO: 2, SEQ ID NO: 4 or SEQ ID NO : 6.
A66. The nucleic acid molecule of clause A64 further comprising a vector nucleic acid sequence.
A67. A host cell which contains the nucleic acid molecule of clause A64.
A68. The host cell of clause A66 which is a mammalian host cell.
A69. An isolated nucleic acid molecule selected from the group consisting of : a) a nucleic acid molecule comprising a nucleotide sequence which is at least 96% identical to the nucleotide sequence of SEQ ID NO : 7, SEQ ID NO : 9 or SEQ ID NO: 11 ; b) a nucleic acid molecule comprising the nucleotide sequence of SEQ ID NO : 7, SEQ ID NO : 9 or SEQ ID NO : 11; c) a nucleic acid molecule which hybridizes to the nucleotide sequence of SEQ ID NO : 7 under stringent conditions.
A70. An isolated nucleic acid molecule which encodes a polypeptide comprising the amino acid sequence of SEQ ID NO: 8, SEQ ID NO: 10 or SEQ ID NO: 12.
A71. The nucleic acid molecule of clause A69 further comprising a vector nucleic acid sequence.
A72. A host cell which contains the nucleic acid molecule of clause A69.
A73. The host cell of clause A72 which is a mammalian host cell.
A74. An isolated polypeptide comprising the amino acid sequence of SEQ ID NO : 2, SEQ ID NO: 4, or SEQ ID NO : 6.
A75. The polypeptide of clause A74, wherein the polypeptide comprises the amino acid sequence of SEQ ID NO : 2.
A76. An isolated polypeptide comprising the amino acid sequence of SEQ ID NO : 8, SEQ ID NO : 10, or SEQ ID NO : 12.
A77. The polypeptide of clause A76, wherein the polypeptide comprises the amino acid sequence of SEQ ID NO: 8.
A78. A method for producing a polypeptide, the method comprising culturing the host cell of clause A67 under conditions in which the nucleic acid molecule is expressed.
A79. A method for producing a polypeptide, the method comprising culturing the host cell of clause A72 under conditions in which the nucleic acid molecule is expressed.
A80. The isolated pathogenic immunoglobulin of clause A22, wherein the immunoglobulin has a light chain variable region comprising the amino acid sequence shown in SEQ ID NO: 12 and the amino acid sequence of SEQ ID NO : 10.
A81. The isolated pathogenic immunoglobulin of clause A22, wherein the immunoglobulin has a heavy chain variable region comprising the amino acid sequence shown in SEQ ID NO : 4 and the amino acid sequence of SEQ ID NO : 6.

## Claims

1. A method for treating or preventing immunoglobulin-mediated reperfusion or ischemic injury, in a subject, preferably a mammal, more preferably a human, comprising:
administering to the subject an effective amount of an inhibitor of an interaction between a pathogenic immunoglobulin and an ischemia-specific antigen or a component of the complement pathway; thereby reducing the number of the pathogenic immunoglobulins bound to the ischemia-specific antigen or which activate complement activity, such that the injury is inhibited or reduced.

2. The method of claim 1, wherein the pathogenic immunoglobulin is an IgM, or is a subclass of IgMs, or is produced by a subpopulation of B cells.

3. The method of claim 1, wherein the ischemia-specific antigen is present on the surface of an endothelial cell or parenchymal tissue.

4. The method of claim 1, wherein the reperfusion or ischemic injury results following a naturally occurring episode, or wherein the reperfusion or ischemic injury occurs during or following a surgical procedure, preferably wherein the surgical procedure is selected from the group consisting of angioplasty, stenting procedure, atherectomy, and bypass surgery.

5. The method of claim 1, wherein the inhibitor is selected from the group consisting of a protein, a peptide, a small organic molecule, an antibody or a fragment thereof, a carbohydrate and a glycoprotein.

6. A method for treating or preventing immunoglobulin-mediated reperfusion or ischemic injury in a subject, preferably a mammal, more preferably a human, comprising :
removing from the subject or inactivating a pathogenic immunoglobulin or a B cell producing the pathogenic immunoglobulin, thereby reducing the amount of the pathogenic immunoglobulin or B cells present in the subject.

7. The method of claim 6, wherein the removing or inactivating step is performed ex vivo; preferably wherein the removing or inactivating step of the pathogenic immunoglobulin is performed by administering to the subject an antiidiotypic antibody; more preferably wherein the removing or inactivating step of the B cell is performed by administering to the subject a B cell targeting moiety coupled to a toxin.

8. The method of claim 1 or 6, wherein the injury occurs in a cardiovascular tissue.

9. An isolated pathogenic immunoglobulin, or antigen binding portion thereof, having one or more of the following properties: (i) is capable of interacting with an ischemia-specific antigen; (ii) is capable of fixing complement; or (iii) is produced by a subpopulation of B cells;
optionally wherein the immunoglobulin is an IgM.

10. A modified pathogenic immunoglobulin having a mutation that alters complement binding or activity; optionally which is capable of interacting with an ischemia-specific antigen; and optionally which is an IgM.

11. A method for isolating an ischemia-specific antigen, comprising:
providing a pathogenic immunoglobulin;
contacting a sample containing the ischemia-specific antigen with the pathogenic immunoglobulin under conditions that allow specific binding of the pathogenic immunoglobulin to the sample;
detecting any changes in the level of binding of the pathogenic antibody to the sample relative to a control, wherein a change in the level of binding of the pathogenic immunoglobulin in the presence of the sample relative to that detected in the control is indicative of specific binding; and
isolating the ischemia-specific antigen from the sample;
optionally a) wherein the sample is enriched in the ischemia specific antigen; or b) wherein the sample is obtained from a subject with reperfusion or ischemic injury; or c) wherein the sample is a biochemical isolate; or d) wherein the sample is an expression library.

12. A method for identifying an inhibitor of an interaction between a pathogenic immunoglobulin and an ischemia-specific antigen or a component of the complement pathway from a plurality of test compounds, comprising :
providing a reaction mixture which includes the pathogenic immunoglobulin and the ischemia-specific antigen or the component of the complement pathway under conditions that allow binding of the pathogenic immunoglobulin and the ischemia-specific antigen or the component of the complement pathway to occur;
contacting the pathogenic immunoglobulin and the ischemia-specific antigen or the component of the complement pathway with one or more test compounds, and
detecting any changes in binding of the pathogenic immunoglobulin and the ischemia-specific antigen or the component of the complement pathway in the presence of a given test compound relative to that detected in the absence of the test compound,
wherein a change in the level of binding between the pathogenic immunoglobulin and the ischemia-specific antigen or the component of the complement pathway in the presence of the test compound relative to that detected in the absence of the test compound indicates that the test compound is an inhibitor of the interaction between the pathogenic immunoglobulin and the ischemia-specific antigen or the component of the complement pathway.

13. The method of claim 12, wherein:
the pathogenic immunoglobulin is a pathogenic IgM; or
wherein the ischemia-specific antigen is obtained from an endothelial tissue or an endothelial lysate; or
wherein the method is performed in vitro; or
wherein the pathogenic immunoglobulin is labeled with a detectable signal; or
wherein the ischemia-specific antigen is labeled with a detectable signal; or
further comprising repeating at least one step; or
wherein the plurality of test compounds, comprises at least 10,10²,10³,10⁴,10⁵,10⁶,10⁷, or 10⁸ compounds; or
wherein the test compound is a peptide or a small organic molecule.

14. A method for treating or preventing immunoglobulin-mediated reperfusion or ischemic injury, comprising :
administering to a subject an agent having a property of inhibiting an interaction between a pathogenic immunoglobulin and an ischemia-specific antigen or a component of the complement pathway as determined by the method of claim 12.

15. The isolated pathogenic immunoglobulin of claim 9, which has a light chain variable region comprising the amino acid sequence of SEQ IID NO : 8; or
which has a heavy chain variable region comprising the amino acid sequence of SEQ ID NO : 2; or
which has a light chain variable region comprising a CDR1 region comprising the amino acid sequence shown in SEQ ID NO : 10; or
which has a light chain variable region comprising a CDR2 region comprising the amino acid sequence shown in SEQ ID NO : 12; or
which has a heavy chain variable region comprising a CDR1 region comprising the amino acid sequence of SEQ ID NO : 4; or
which has a heavy chain variable region comprising a CDR2 region comprising the amino acid sequence shown in SEQ ID NO : 6.

16. The isolated pathogenic immunoglobulin of claim 9, wherein the immunoglobulin is a recombinant antibody; or is a human immunoglobulin; or wherein the immunoglobulin is a non-human immunoglobulin, preferably from a mammal, more preferably wherein the mammal is selected from the group consisting of cow, goat, mouse, rat, sheep, pig, and rabbit.

17. The isolated pathogenic immunoglobulin of claim 9, wherein the immunoglobulin comprises the heavy chain CDR1 and CDR2 regions shown in SEQ ID NO : 4 and SEQ ID NO : 6, or antigen binding fragments thereof, and the light chain CDR1 and CDR2 regions shown in SEQ ID NO : 10 and SEQ ID NO : 12, or antigen binding fragments thereof; preferably wherein the immunoglobulin comprises human framework regions.

18. An isolated nucleic acid molecule selected from the group consisting of:
a) a nucleic acid molecule comprising a nucleotide sequence which is at least 96% identical to the nucleotide sequence of SEQ ID NO : 1, SEQ ID NO : 3 or SEQ ID NO : 5; or SEQ ID NO : 7, SEQ ID NO : 9, or SEQ ID NO : 11 ;
b) a nucleic acid molecule comprising the nucleotide sequence of SEQ ID NO: 1, SEQ ID NO : 3 or SEQ ID NO : 5; or SEQ ID NO : 7, SEQ ID NO : 9, or SEQ ID NO: 11; and
c) a nucleic acid molecule which hybridizes to the nucleotide sequence of SEQ ID NO: 1 or SEQ ID NO : 7 under stringent conditions.

19. An isolated nucleic acid molecule which encodes a polypeptide comprising the amino acid sequence of SEQ ID NO : 2, SEQ ID NO : 4 or SEQ ID NO : 6; or SEQ ID NO : 8, SEQ ID NO: 10 or SEQ ID NO: 12.

20. The nucleic acid molecule of claim 18 further comprising a vector nucleic acid sequence.

21. A host cell, preferably a mammalian host cell, which contains the nucleic acid molecule of claim 18.

22. An isolated polypeptide comprising the amino acid sequence of SEQ ID NO : 2, SEQ ID NO : 4, or SEQ ID NO : 6; or SEQ ID NO : 8, SEQ ID NO: 10 or SEQ ID NO: 12.

23. A method for producing a polypeptide, the method comprising culturing the host cell of claim 21 under conditions in which the nucleic acid molecule is expressed.

24. The isolated pathogenic immunoglobulin of claim 9, wherein the immunoglobulin has a light chain variable region comprising the amino acid sequence shown in SEQ ID NO: 12 and the amino acid sequence of SEQ ID NO : 10; or wherein the immunoglobulin has a heavy chain variable region comprising the amino acid sequence shown in SEQ ID NO : 4 and the amino acid sequence of SEQ ID NO : 6.
